# EUROPEAN PATENT APPLICATION

(11) **EP 4 227 414 A1**
(43) Date of publication of application: **16.08.2023**
(21) Application number: 21879354.5
(22) Date of filing: 12.10.2021
(51) Int. Cl.: C12N 15/63, A61K 48/00, A61P 43/00

(54) **NOVEL PIGGYBAC TRANSPOSON SYSTEM AND USE THEREOF**

(30) Priority: 12.10.2020 CN 202011085981
(71) Applicant: Shanghai Juncell Therapeutics Co., Ltd., Shanghai 201800 (CN)
(72) Inventor: JIN, Huajun, Shanghai 201800 (CN); XU, Fuhui, Shanghai 201800 (CN); HUANG, Chen, Shanghai 201800 (CN); MA, Xingming, Shanghai 201800 (CN); LIU, Tianyi, Shanghai 201800 (CN); GUO, Xiaochun, Shanghai 201800 (CN)
(74) Representative: Nederlandsch Octrooibureau
(86) International application number: PCT/CN2021/123191
(87) International publication number: WO 2022/078310

(57) **Abstract**

Provided is a mucleic acid construct, comprising the following elements: a transposon 3' terminal repeat, first poly Asequence, an insulator sequence having a transcription termination function, a transposon 5' terminal repeat, a transposase coding sequence, and a promoter that controls the expression of the transposase. Also provided are a host cell or pharmaceutical composition comprising said nucleic acid construct, and a use thereof.

## Description

### Technical Field

The invention relates to the field of transposon vectors, in particular to a novel PiggyBac transposon system and its application.

### Background

It is a necessary condition for the realization of many cell therapy technologies, including immune cell therapy, to introduce exogenous genes into the target cells for stable expression so as to realize the transgenic transformation of the target cells. Compared with transient expression, stably expressed exogenous genes can be integrated into the genome of target cells, and can maintain stable expression for a long time even in the case of multiple cell passages or changes in culture conditions. Commonly used transgenic systems include viral-based vectors, eukaryotic expression plasmid vectors, and transposon vectors. Genetic modification of primary human T cells using non-viral vector-based approaches has proven to be extremely difficult. Therefore, worldwide, most laboratories are still using viral vector systems for transgenic modification of cells, including retroviral vectors, such as lentiviral vector systems. Although the virus vector system has been widely used, there are insurmountable problems such as complex virus preparation operations, relatively high safety risks, and high production costs.

In recent years, transposon vector systems have been increasingly used to modify immune cells for tumor immunotherapy. The earliest transposon used in mammals was the "Sleeping Beauty" transposon from fish, but it has defects such as the overproduction inhibition effect and small insert fragments (about 5kb), etc. The application in transgenic manipulation is severely limited. PiggyBac transposon is another type of transposon system derived from Lepidoptera insects, which can carry larger fragments and can integrate into a variety of eukaryotic host cells. The PiggyBac (PB) transposon system mainly transposes through the "cut-paste" mechanism, and does not leave a footprint at the original site after transposition, and is increasingly used after modification in genome research, gene therapy, cell therapy, stem cell induction and induced differentiation, etc.

WO2019046815A1 discloses a traditional PiggyBac transposon-based binary system, which includes a vector containing PiggyBac transposase and a helper vector containing 5'ITR and 3'ITR. The PiggyBac transposon system combined with electroporation can introduce exogenous genes into T cells, NK cells and HSPCs. However, the binary system requires the PiggyBac transposase vector and the helper vector to be transfected into the cell simultaneously before transposition can occur, which is both demanding and difficult for transfection. Meanwhile, the mechanism of the PiggyBac transposition system, PiggyBac transposase inserting transposed fragments into the genome through the "cut-paste" mechanism, is reversible, so as long as the expression of PiggyBac enzyme continues , the transposed fragments that have been integrated into the genome may also be re-cut, resulting in genome instability and substantially reducing the transposition efficiency. The transposition efficiency of the common PiggyBac binary transposition system in T cells is usually around 10%, which is relatively low. Moreover, WO2019046815A1 also describes that plasmid DNA is highly toxic to T cells, and its toxicity to T cells is related to the amount of DNA used for electroporation. The binary system undoubtedly requires a larger amount of plasmid DNA for electroporation, increases the toxicity to cells, especially T cells, and reduces the viability of T cells transfected with plasmid DNA.

CN105154473B discloses a unary PiggyBac transposon vector, which combines the PiggyBac transposase vector and the helper vector in the traditional binary PiggyBac transposition system into one vector, and through a mechanism in which the expression cassette of the PiggyBac transposase and the exogenous gene expression cassette share the same bidirectional polyA sequence in a single expression vector and the polyA in the PiggyBac transposase expression cassette is cut off and self-inactivated after integration, the continuous expression of the constitutive PiggyBac transposase was effectively reduced, and the transposition efficiency of PiggyBac transposase was improved. In the meantime, the binary system is reduced to a single vector, which greatly lowers the total amount of DNA and decreases the toxicity of exogenous DNA to T cells. However, the expression cassette of the PiggyBac transposase and that of the exogenous gene sharing the same bidirectional polyA sequence will cause mutual influence between the two expression cassettes opposite in direction, and in some types of cells, the integration efficiency of this unary transposon vector still needs further improvement.

Therefore, there is still a lack of an higly efficient unary transposon system that can efficiently integrate exogenous genes while shutting down the integration function in time.

### Summary

The inventors constructed an integration system based on the PiggyBac transposon, which can mediate the efficient integration and stable expression of exogenous genes in host cells.

One aspect of the present invention relates to a nucleic acid construct, which comprises or consists of the following elements: a transposon 3' terminal repeat sequence, first polyA sequence, an insulator sequence with transcription termination function, a transposon 5' terminal repeat sequence . In one or more embodiments, the nucleic acid construct further comprises one or more elements selected from the group consisting of: a transposase coding sequence, a promoter controlling the expression of the transposase, a multiple cloning insertion site, an enhancer, 5'UTR, a second polyA sequence and a exogenous gene of interest.

The present invention further provides a nucleic acid construct, which comprises the following elements: a transposon 3' terminal repeat sequence, a first polyA sequence, an insulator sequence with transcription termination function, a transposon 5' terminal repeat sequence, a transposase encoding sequence and a promoter controlling the expression of the transposase.

In one or more embodiments, the nucleic acid construct further comprises one or more elements selected from the group consisting of a multiple cloning insertion site, an enhancer, a 5'UTR, a second polyA sequence and an exogenous gene of interest.

In one or more embodiments, any one or more of the transposase coding sequence, the promoter controlling the expression of the transposase, the 5'UTR and the second polyA sequence are outside the region between the transposon 3' terminal repeat and the transposon 5' terminal repeat.

In one or more embodiments, the nucleic acid construct comprises sequentially : a transposon 3' terminal repeat sequence, a first polyA sequence, an insulator sequence with transcription termination function, a transposon 5' terminal repeat sequence, a promoter for controlling transposase expression, a transposase coding sequence and a second polyA sequence.

In one or more embodiments, the nucleic acid construct comprises sequentially: a transposon 3' terminal repeat sequence, a multiple cloning insertion site, a first polyA sequence, an insulator sequence with transcription termination function, a transposon 5' terminal repeat sequence, a transposase coding sequence and a promoter for controlling transposase expression.

In one or more embodiments, the nucleic acid construct comprises sequentially: a transposon 3' terminal repeat sequence, a multiple cloning insertion site, a first polyA sequence, an insulator sequence with transcription termination function, a transposon 5' terminal repeat sequence, a promoter for controlling transposase expression, a transposase coding sequence and a second polyA sequence.

In one or more embodiments, the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a transposase coding sequence and a promoter for controlling transposase expression.

In one or more embodiments, the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a promoter for controlling transposase expression, a transposase coding sequence and a second polyA sequence.

In one or more embodiments, the nucleic acid construct comprises sequentially: a transposon 3' terminal repeat sequence, a multiple cloning insertion site, a first polyA sequence, an insulator sequence with transcription termination function, a transposon 5' terminal repeat sequence, a transposase coding sequence, a 5'UTR and a promoter for controlling transposase expression.

In one or more embodiments, the nucleic acid construct comprises sequentially: a transposon 3' terminal repeat sequence, a multiple cloning insertion site, a first polyA sequence, an insulator sequence with transcription termination function, a transposon 5' terminal repeat sequence, a promoter for controlling transposase expression, a 5'UTR, a transposase coding sequence and a second polyA sequence.

In one or more embodiments, the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a transposase coding sequence, a 5'UTR and a promoter for controlling transposase expression.

In one or more embodiments, the nucleic acid construct comprises sequentially: a transposon 3' terminal repeat sequence, a multiple cloning insertion site, first polyA sequence, an enhancer, an insulator sequence with transcription termination function, a transposon 5' terminal repeat sequence, a promoter for controlling transposase expression, 5'UTR, a transposase coding sequence and a second polyA sequence.

In one or more embodiments, the nucleic acid construct comprises sequentially: a transposon 3' terminal repeat sequence, an insulator sequence with a transcription termination function, a multiple cloning insertion site, a first polyA sequence, a transposon 5' terminal repeat sequence, a promoter for controlling transposase expression, a 5'UTR, a transposase coding sequence and a second polyA sequence.

In one or more embodiments, the nucleic acid construct comprises sequentially: a transposon 3' terminal repeat sequence, an insulator sequence with a transcription termination function, a multiple cloning insertion site, a first polyA sequence, an enhancer, a transposon 5' terminal repeat sequence, a promoter for controlling transposase expression, a 5'UTR, a transposase coding sequence and a second polyA sequence.

In one or more embodiments, the nucleic acid construct comprises in turn: a transposon 3' terminal repeat sequence, an enhancer, an insulator sequence with a transcription termination function, a multiple cloning insertion site, a first polyA sequence, a transposon 5' terminal repeat sequence, a promoter for controlling transposase expression, a 5'UTR, a transposase coding sequence and a second polyA sequence.

In one or more embodiments, the multiple cloning insertion site is used to be inserted operably with the coding sequence of the exogenous gene and optionally the promoter controlling the expression of the exogenous gene.

In one or more embodiments, the orientation of the tailing signal function of the first polyA sequence and the second polyA sequence is the same or opposite.

In one or more embodiments, the orientation of the expression cassette of the transposase is the same as or opposite to that of the exogenous gene.

In one or more embodiments, the orientation of the expression cassette of the transposase is the same as or opposite to the orientation of the sequence between the transposon 3' terminal repeat and the transposon 5' terminal repeat.

In one or more embodiments, each of the aforementioned elements is independently in single copy or multiple copies.

In one or more embodiments, each of the above-mentioned elements is connected directly or through a linker or restriction site.

The nucleic acid construct according to any embodiment of the present invention, wherein the positions of the transposon 5' terminal repeat sequence and the transposon 3' terminal repeat sequence can be exchanged.

In one or more embodiments, the transposon 3' terminal repeat is a PiggyBac transposon 3' terminal repeat. In a preferred embodiment, the nucleotide sequence of the transposon 3' terminal repeat sequence is as shown in SEQ ID NO:1.

In one or more embodiments, the sequence of the multiple cloning insertion site is as shown in SEQ ID NO:2.

In one or more embodiments, the first polyA sequence is as shown in SEQ ID NO:3, 13 or 16.

In one or more embodiments, the second polyA sequence is as shown in SEQ ID NO:3, 13 or 16.

In one or more embodiments, the enhancer is selected from the group consisting of: CMV enhancer sequence, SV40 enhancer, human epsilon globin 5' HS2 enhancer, chicken beta globin gene 5' HS4 enhancer. Preferably, the enhancer sequence is as shown in any one of SEQ ID NO:4, 26-28.

In one or more embodiments, an insulator sequence with a transcription termination function is as shown in SEQ ID NO:5 or 15.

In one or more embodiments, the transposon 5' terminal repeat is a PiggyBac transposon 5' terminal repeat. In a preferred embodiment, the nucleotide sequence of the transposon 5' terminal repeat sequence is as shown in SEQ ID NO:6.

In one or more embodiments, the transposase is PiggyBac transposase. In one or more embodiments, the amino acid sequence of the PiggyBac transposase is as shown in SEQ ID NO:36; preferably, the coding sequence of the PiggyBac transposase is as shown in SEQ ID NO:7.

In one or more embodiments, the 5'UTR sequence is selected from the 5'UTR of C3 gene, ORM1 gene, HPX gene, FGA gene, AGXT gene, ASL gene, APOA2 gene, ALB gene. Preferably, the 5'UTR sequence is as shown in any one of SEQ ID NO:8, 17-24.

In one or more embodiments, the promoter is selected from: CMV promoter, miniCMV promoter, CMV53 promoter, miniSV40 promoter, miniTK promoter, MLP promoter, pJB42CAT5 promoter, YB_TATA promoter, EF1α promoter, SV40 promoter, UbiquitinB promoter, CAG promoter, HSP70 promoter, PGK-1 promoter, β-actin promoter, TK promoter and GRP78 promoter. Preferably, the promoter is selected from the group consisting of miniCMV promoter, CMV53 promoter, miniSV40 promoter, miniTK promoter, MLP promoter, pJB42CAT5 promoter and YB_TATA. In one or more embodiments, the promoter sequence is as shown in any one of SEQ ID NO:9, 37-42. Preferably, the promoter is a miniCMV promoter, the sequence of which is as shown in SEQ ID NO:9.

In one or more embodiments, the transposase coding sequence contains or is operably linked to a single copy or multiple copies of a nuclear localization signal coding sequence. In one or more embodiments, the nuclear localization signal is a c-myc nuclear localization signal, preferably having the sequence shown in SEQ ID NO:35.

In one or more embodiments, the nucleic acid construct comprises the sequence shown in SEQ ID NO:10 or 14.

In one or more embodiments, the nucleic acid construct is a recombinant vector.

In one or more embodiments, the nucleic acid construct is a recombinant cloning vector or a recombinant expression vector.

The present invention further provides a host cell comprising: (1) the nucleic acid construct described in any embodiment herein, and/or (2) the sequence between the transposon 3' terminal repeat and the transposon 5' terminal repeat of the nucleic acid construct described in any embodiment herein.

In one or more embodiments, the host cell is a mammalian cell.

In one or more embodiments, the host cells are selected from immune cells, Jurkat cells, K562 cells, embryonic stem cells, tumor cells, HEK293 cells and CHO cells.

In one or more embodiments, the immune cells are any one or more selected from the group consisting of T cells, B cells, CIK cells, LAK cells, NK cells, cytotoxic T lymphocytes (CTL), dendritic cells (DC), tumor infiltrating lymphocytes (TIL), macrophages, NK T cells, and γδT cells.

The present invention further provides a pharmaceutical composition, comprising the nucleic acid construct or host cell described in any embodiment herein and a pharmaceutically acceptable excipient.

The present invention also provides the use of the nucleic acid construct or host cell described in any embodiment herein in the manufacture of or as a medicament, reagent or tool for integrating an exogenous gene expression cassette into a target cell genome, or for gene therapy, cell therapy, stem cell induction or differentiation.

In one or more embodiments, the target cells are mammalian cells.

In one or more embodiments, the target cells are selected from the group consisting of T cells, Jurkat cells, K562 cells, embryonic stem cells, tumor cells, HEK293 cells and CHO cells.

The present invention further provides a method for integrating an exogenous gene or its expression cassette into the genome of a cell, comprising introducing the nucleic acid construct described in any embodiment herein containing the exogenous gene and optionally its promoter into the cells, and optionally incubating the said cells under conditions in which the transposase integrates the exogenous gene or its expression cassette into the genome of the cell.

In one or more embodiments, the transposase is PiggyBac transposase.

In one or more embodiments, the introduction includes virus-mediated transformation, microinjection, particle bombardment, biolistic transformation, electroporation, and the like. In one embodiment of the invention, the introduction is electroporation.

In one or more embodiments, the cells are incubated for at least three passages.

The present invention further provides a cell obtained by the method described herein in which exogenous genes or their expression cassettes are integrated in the genome.

### Brief Description of Figures

Figures 1A and 1B, schematic representations of the nucleic acid constructs described herein.
Fig. 2. Fluorescent images of Jurkat cells electrotransfected with pKB20-EGFP, pKB201-EGFP, and pKB202-EGFP, respectively.
Fig. 3. The results of flow cytometry assay of Jurkat cells electrotransfected with pKB20-EGFP, pKB201-EGFP, and pKB202-EGFP, respectively.
Fig. 4. The number of viable cells of Jurkat cells electrotransfected with pKB20-EGFP, pKB201-EGFP, and pKB202-EGFP, respectively.
Figure 5, the expression levels of PB transposase in Jurkat cells electrotransfected with pKB20-EGFP, pKB201-EGFP and pKB202-EGFP, respectively.
Fig. 6. Fluorescent images of K562 cells electrotransfected with pKB20-EGFP, pKB201-EGFP, pKB202-EGFP and pKC20-EGFP, respectively.Fig. 7. The number of viable cells of K562 cells electrotransfected with pKB20-EGFP, pKB201-EGFP, pKB202-EGFP and pKC20-EGFP, respectively.
Fig. 8. The results of flow cytometry of K562 cells electrotransfected with pKB20-EGFP, pKB201-EGFP and pKB202-EGFP, respectively.
Fig. 9. The fluorescence positive proportion of K562 cells electrotransfected with pKB20-EGFP, pKB201-EGFP, and pKB202-EGFP, respectively.
Fig. 10. Fluorescent images of primary T cells electrotransfected with pKB20-EGFP, pKB201-EGFP, pKB202-EGFP and pKC20-EGF, respectively.
Fig. 11. The results of flow cytometry assay of T cells electrotransfected with pKB20-EGFP, pKB201-EGFP and pKB202-EGFP, respectively.
Figure 12, the results of the positive proportion of electrotransfection of Jurkat cells using the dual-plasmid PB transposition system.
Figure 13, the results of the positive proportion of electrotransfection of T cells using the dual-plasmid PB transposition system.
Fig. 14, the positive proportion of Jurkat cells electrotransfected with pKB20-EGFP with reduced amount of plasmid.
Figure 15, the time-course change of the residual copy number of the vector in cells.
Fig. 16, the positive proportion of Jurkat cells electrotransfected with pKB2003-EGFP.
Fig. 17, the positive proportion of primary T cells electrotransfected with pKB205-EGFP.
Figure 18. Schematic diagram of pKB20 vector-mediated genomic integration sites in K562 sample 1.
Figure 19. Schematic diagram of pKB20 vector-mediated genomic integration sites in K562 sample 2.
Figure 20. Schematic diagram of pKB20 vector-mediated genomic integration sites in Jurkat sample 1.
Figure 21. Schematic diagram of pKB20 vector-mediated genomic integration sites in Jurkat sample 2.
Figure 22, flow cytometric results of the positive proportion of primary T cells electrotransfected with pKB20-HER2CAR.Figure 23, the results of in vitro RTCA killing of target cells SKOV-3 by HER2CAR-T cells.
Figure 24, flow cytometric results of the positive proportion of primary T cells electrotransfected with pKB20-NY-ESO-1 TCR.
Figure 25, the in vitro RTCA killing results of NY-ESO-1 TCR-T on target cell A3 75.
Fig. 26, flow cytometric results of the positive proportion of K562 cells electrotransfected with pNB328-EGFP.
Fig. 27, flow cytometric results of the positive proportion of primary T cells electrotransfected with pNB328-EGFP.

### Detailed Description

It should be understood that within the scope of the present invention, the above-mentioned technical features of the present invention and the technical features specifically described in the following (such as the examles) can be combined with each other to form a preferred technical solution.

As used herein and in the appended claims, the singular forms "a", "an" and "the" include plural referents unless the context clearly dictates otherwise. Also, the terms "a" (or "an"), "one or more" and "at least one" are used interchangeably herein. The term "comprises" and variations thereof, where these terms appear in the specification and claims, do not have a limiting meaning. Accordingly, the terms "comprises," "comprising," and "containing" are used interchangeably.

### Nucleic acid constructs and cells

The term "nucleic acid construct", defined herein as a single- or double-stranded nucleic acid molecule, preferably refers to an artificially constructed nucleic acid molecule. Optionally, the nucleic acid construct further comprises one or more operably linked regulatory sequences, which can direct the expression of the coding sequence in a suitable host cell under compatible conditions. Expression is to be construed as comprising any step involved in the production of a protein or polypeptide, including, but not limited to, transcription, post-transcriptional modification, translation, post-translational modification, and secretion. The transposition system described herein is preferably a unary nucleic acid construct, that is, one nucleic acid construct can achieve high-efficiency transposition.

In the present invention, unless otherwise specified, the orientation of the transposase expression cassette is defined as the reverse orientation. The orientation and/or order referred to as "sequentially" in the above "sequentially comprising the following elements" refers to an upstream to downstream orientation. In the present invention, unless otherwise specified, the orientation along the above-mentioned "forward" is from upstream to downstream, and the orientation along the above-mentioned "reverse" is from downstream to upstream.

In the present invention, the term "expression cassette" refers to the complete set of elements required to express a gene, including promoter, gene coding sequence, and PolyA tailing signal sequence.

The term "operably inserted/linked" is defined herein as a conformation in which a regulatory sequence is located in an appropriate position relative to the coding sequence of a DNA sequence such that the regulatory sequence directs the expression of a protein or polypeptide. In the nucleic acid construct of the present invention, the multiple cloning site is operably inserted into one or more same or different exogenous genes and optionally a promoter controlling the expression of the exogenous gene through DNA recombination technology, or its multiple cloning sites are replaced with one or more same or different coding sequences of the exogenous gene and optionally a promoter controlling the expression of the exogenous gene. The "operably linked" can be achieved by means of DNA recombination, specifically, the nucleic acid construct is a recombinant nucleic acid construct.

The "exogenous gene" mentioned herein can be a nucleic acid molecule from any source, which is expressed or functions after being transposed into the host cell genome. Non-limiting examples of exogenous genes include luciferin reporter genes (e.g., green fluorescent protein, yellow fluorescent protein, etc.), luciferase genes (e.g., firefly luciferase, renilla luciferase, etc.), native functional protein genes, RNAi genes and artificial chimeric genes (such as chimeric antigen receptor genes, Fc fusion protein genes, full-length antibody genes).

The term "coding sequence" is defined herein as that portion of a nucleic acid sequence which directly specifies the amino acid sequence of its protein product. The boundaries of the coding sequence are generally determined by the ribosome binding site (for prokaryotic cells) immediately upstream of the 5' open reading frame of the mRNA and the transcription termination sequence immediately downstream of the 3' open reading frame of the mRNA. A coding sequence may include, but is not limited to, DNA, cDNA, and recombinant nucleic acid sequences.

The term "regulatory sequence" is defined herein to include all components necessary or advantageous for the expression of the peptides of the invention. Each regulatory sequence may be native or foreign to the nucleic acid sequence encoding the protein or polypeptide. These regulatory sequences include, but are not limited to, a leader sequence, polyA sequence, propeptide sequence, promoter, signal sequence, and transcription terminator. At a minimum, regulatory sequences will include a promoter and termination signals for transcription and translation. The control sequences with linkers may be provided for the purpose of introducing specific restriction sites for ligation of the control sequences with the coding region of the nucleic acid sequence encoding a protein or polypeptide.

The control sequence may be a suitable promoter sequence, which is a nucleic acid sequence recognized by the host cell in which the nucleic acid sequence is expressed. The promoter sequence contains transcriptional regulatory sequences that mediate the expression of the protein or polypeptide. The promoter sequence is usually operably linked to the coding sequence of the protein to be expressed. The promoter can be any nucleotide sequence that shows transcriptional activity in the host cell of choice, including mutated, truncated, and hybrid promoters, and can be derived from genes encoding extracellular or intracellular polypeptides homologous or heterologous to the host cell.

The regulatory sequence may also be a suitable transcription termination sequence, a sequence recognized by a host cell to terminate transcription. A termination sequence is operably linked to the 3' end of a nucleic acid sequence encoding a protein or polypeptide. Any terminator that is functional in the host cell of choice may be used in the present invention.

The regulatory sequence may also be a suitable leader sequence, an untranslated region of an mRNA important for translation by the host cell. A leader sequence is operably linked to the 5' end of a nucleic acid sequence encoding a polypeptide. Any terminator that is functional in the host cell of choice may be used in the present invention.

The control sequence can also be a signal peptide coding region, which codes an amino acid sequence connected to the amino terminal of the protein or polypeptide, and can guide the coded polypeptide into the secretory pathway of the cell. The 5' end of the coding region of the nucleic acid sequence may naturally contain a signal peptide coding region naturally linked in translation reading frame with the segment of the coding region of the secreted polypeptide. Alternatively, the 5' end of the coding region may contain a signal peptide coding region foreign to the coding sequence. It may be necessary to add a foreign signal peptide coding region when the coding sequence does not normally contain a signal peptide coding region. Alternatively, the native signal peptide coding region can be simply replaced with a foreign signal peptide coding region to enhance polypeptide secretion. However, any signal peptide coding region that directs the expressed polypeptide into the secretory pathway of the host cell used may be used in the present invention.

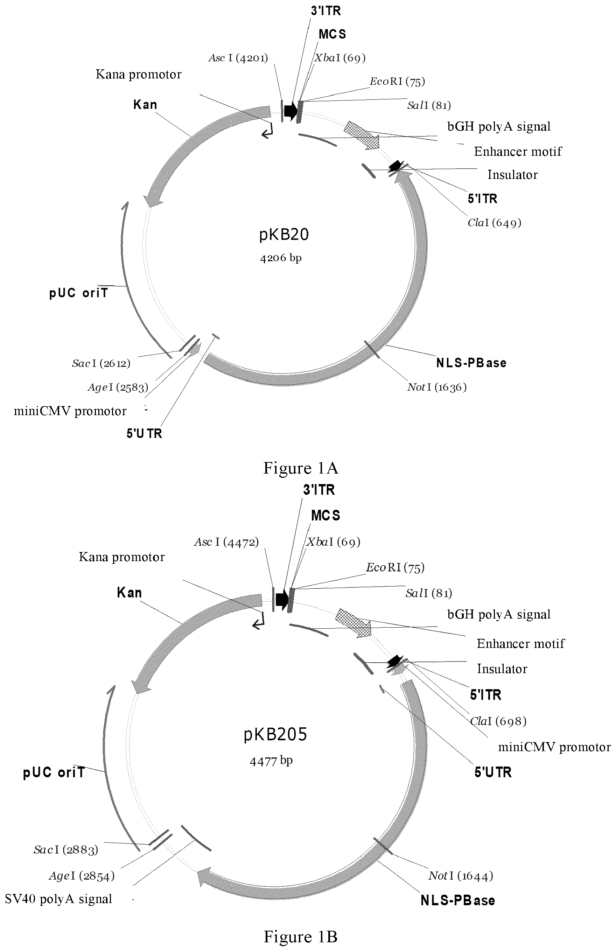

The nucleic acid construct of the present invention comprises the following elements: a transposon 3' terminal repeat sequence, a first polyA sequence, an insulator sequence with transcription termination function, a transposon 5' terminal repeat sequence, a transposase encoding sequence and a promoter controlling the expression of the transposase. The nucleic acid construct may also comprise one or more elements selected from the group consisting of a multiple cloning insertion site, an enhancer, a 5'UTR, and a second polyA sequence. In a particularly preferred embodiments, the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a transposase coding sequence, a 5'UTR and a promoter for controlling transposase expression of the transposase, as shown in Figure 1A. In another particularly preferred embodiment, the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a promoter controlling the expression of the transposase, a 5'UTR, the transposase coding sequence and a second polyA sequence, as shown in Figure 1B. Each element in the nucleic acid construct herein is independently in single or multiple copies.

Herein, the position of the repeat sequence of the 5' end of the transposon and the repeat sequence of the 3' end of the transposon can be interchanged. Preferably, the 5' end repeat sequence of the transposon is the 5' end repeat sequence of the PiggyBac transposon; the 3' end repeat sequence of the transposon is the 3' end repeat sequence of the PiggyBac transposon.

Herein, the transposase is preferably PiggyBac transposase, the coding sequence of which contains or is operably connected with single copy or multiple copies of the nuclear localization signal coding sequence, thereby improving transposition efficiency. An exemplary PiggyBac transposase coding sequence is as shown in SEQ ID NO:7. An exemplary nuclear localization signal coding sequence is as shown in SEQ ID NO:35.

The nucleic acid constructs of the present invention can use polyA sequences for transposases and exogenous genes. This design can shorten the full length of the nucleic acid construct to a certain extent, and is helpful for the incorporation of longer exogenous genes for transposition. Alternatively, the nucleic acid construct of the present invention can also use separate polyA sequences for the transposase and the exogenous gene, and the orientation of the tailing signal function of the two can be the same or opposite. This design avoids the mutual influence between two expression cassette in opposite orientations by sharing the same bidirectional polyA sequence. The polyA sequences described herein may or may not have a bidirectional transcriptional termination function. Preferably, the polyA sequence is independently selected from SEQ ID NO:3, 13 or 16.

The nucleic acid constructs of the present invention can also or further use insulator sequences for transcription termination for transposases and exogenous genes. Therefore, an insulator sequence may be incorporated at either end of any polyA sequence. Preferably, the insulator sequence of the invention is located between the 5' terminal repeat of the transposon and the 3' terminal repeat of the transposon. The insulator sequence described herein has the function of transcription termination, and the sequence may be any sequence known in the art that has the function of transcription termination. Preferably, the insulator sequence with transcription termination function is as shown in SEQ ID NO:5 or 15.

Therefore, transcription termination of the transposase can be achieved by using a polyA sequence, an insulator sequence, or a polyA sequence and an insulator sequence; transcription termination of the exogenous gene can be achieved by using a polyA sequence, an insulator sequence, or a polyA sequence and an insulator sequence. Preferably, any of said insulator sequence are located between said the 5' terminal repeat sequence of the transposon and the 3' terminal repeat sequence of the transposon.

In the nucleic acid construct of the present invention, a suitable promoter sequence for the transposase is a promoter sequence capable of driving high-level expression of the transposase operably linked thereto, including but not limited to the early stage of simian virus 40 (SV40) promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Rous sarcoma virus promoters, as well as human gene promoters, such as, but not limited to, actin promoter, myosin promoter, heme promoter, and creatine kinase promoter. In one or more embodiments, the promoter of the transposase is selected from: CMV promoter, miniCMV promoter, CMV53 promoter, miniSV40 promoter, miniTK promoter, MLP promoter, pJB42CAT5 promoter, YB_TATA promoter, EF1α promoter, SV40 promoter, UbiquitinB promoter, CAG promoter, HSP70 promoter, PGK-1 promoter, β-actin promoter, TK promoter and GRP78 promoter. Preferably, the promoter is selected from the group consisting of miniCMV promoter, CMV53 promoter, miniSV40 promoter, miniTK promoter, MLP promoter, pJB42CAT5 promoter and YB_TATA promoter. More preferably, the promoter is a miniCMV promoter. The miniCMV is much shorter than the CMV promoter, making the vector smaller and more conducive to the integration of larger exogenous genes. In one or more embodiments, a 5'UTR sequence is added between the miniCMV and the transposase to enhance transcription and translation. The 5'UTR sequence is as shown in any one of SEQ ID NO:8, 17-24.

A nucleic acid construct of the invention may contain an enhancer, which may be located at either end of any element in a nucleic acid construct described herein other than an enhancer. Preferably, the enhancer is located between the 3' terminal repeat of the transposon and the 5' terminal repeat of the transposon. More preferably, the enhancer is located downstream of the first polyA sequence. The enhancer sequence is as shown in any one of SEQ ID NO:4, 25-28. Herein, the nucleic acid construct may contain neither the 5'UTR sequence nor the enhancer sequence, or contain eitheror both, and the resulting nucleic acid construct can efficiently integrate the exogenous gene into the cell genome.

It may also be desirable to add regulatory sequences that regulate expression of the polypeptide according to the growth of the host cell. Examples of regulatory systems are those that switch gene expression on or off in response to chemical or physical stimuli, including in the presence of regulatory compounds. Other examples of regulatory sequences are those that enable gene amplification. In these instances, the nucleic acid sequence encoding the protein or polypeptide should be operably linked to the regulatory sequences.

In a specific embodiment containing one polyA signal sequence, the nucleic acid construct of the present invention sequentially comprises a PiggyBac transposon 3' terminal repeat sequence (3'ITR) (SEQ ID NO:1), multiple cloning site (SEQ ID NO :2), a polyA signal sequence (SEQ ID NO: 3, 13 or 16), optionally an enhancer motif sequence (any in SEQ ID NO: 4, 25-28), an insulator sequence (SEQ ID NO: 5 or 15), the reverse complement sequence of a PiggyBac transposon 5' terminal repeat (5'ITR) (SEQ ID NO:6), the reverse complement sequence of a PiggyBac transposase coding sequence (SEQ ID NO:7), optionally the reverse complement of a 5'UTR sequence (any one of SEQ ID NO: 8, 17-24) and the reverse complement sequence of a miniCMV promoter sequence (SEQ ID NO: 9). In one or more embodiments, the nucleic acid construct of the present invention has the sequence shown in SEQ ID NO:10.

In a specific embodiment containing two polyA signal sequences, the nucleic acid construct of the present invention sequentially comprises a PiggyBac transposon 3' terminal repeat sequence (3'ITR) (SEQ ID NO:1), multiple cloning site (SEQ ID NO :2), a first polyA signal sequence (SEQ ID NO: 3, 13 or 16), optionally an enhancer motif sequence (any in SEQ ID NO: 4, 25-28), an insulator sequence (SEQ ID NO: 5 or 15), a PiggyBac transposon 5' terminal repeat sequence (5'ITR) (SEQ ID NO:6), a miniCMV promoter sequence (SEQ ID NO:9), optionally a 5'UTR sequence (any one of SEQ ID NO: 8, 17-24), a PiggyBac transposase coding sequence (SEQ ID NO:7) and a second polyA signal sequence (SEQ ID NO:3, 13 or 16).. In one or more embodiments, the nucleic acid construct of the present invention has the sequence shown in SEQ ID NO:14.

In certain embodiments, the nucleic acid construct is a recombinant vector. The recombinant vector can be a recombinant cloning vector or a recombinant expression vector. The elements of the nucleic acid constructs of the present invention can be incorporated into many types of vectors, e.g., plasmids, phagemids, phage derivatives, animal viruses, and cosmids. In general, suitable vectors contain an origin of replication functional in at least one organism, a promoter sequence, convenient restriction sites and one or more selectable markers.

The vector introduced into the cells may also contain either or both of a selectable marker gene and a reporter gene for the purpose of assessing the expression of the carried gene to facilitate the identification and selection of cells from a cell population. Selectable markers can be carried on a single DNA fragment and used in co-transfection procedures. Both the selectable marker and the reporter gene may be flanked by appropriate regulatory sequences to enable expression in the host cell. Useful selectable markers include Flag, HA or V5. Reporter genes are used to identify potentially transfected cells and to assess the functionality of regulatory sequences. Expression of the reporter gene is measured at an appropriate time after the DNA has been introduced into the recipient cells. Suitable reporter genes may include genes encoding luciferase, β-galactosidase, chloramphenicol acetyltransferase, secreted alkaline phosphatase, or the green fluorescent protein gene.

Recombinant cloning vectors can be used to provide coding sequences containing the various elements of the nucleic acid constructs of the invention and optionally exogenous genes. The recombinant cloning vector can be a recombinant vector obtained by recombining each element of the nucleic acid construct of the present invention with pUC18, pUC19, pMD18-T, pMD19-T, pGM-T vector, pUC57, pMAX or pDC315 series vectors.

The recombinant expression vector can be used to integrate the exogenous gene expression cassette into the genome and express it through the elements of the nucleic acid construct of the present invention in a suitable host cell. This vector may be suitable for replicating and integrating eukaryotic cells. A typical cloning vector contains transcriptional and translational terminators, initiation sequences and a promoter useful for regulating the expression of the desired nucleic acid sequence. The recombinant expression vector is a recombinant vector obtained by recombining the elements of the nucleic acid construct of the present invention with pCDNA3 series vectors, pCDNA4 series vectors, pCDNA5 series vectors, pCDNA6 series vectors, pRL series vectors, pUC57 vectors, pMAX vectors or pDC315 series vectors;

The recombinant vector (recombinant cloning vector or recombinant expression vector) can be a recombinant viral vector, including but not limited to a recombinant adenoviral vector, a recombinant adeno-associated viral vector, a recombinant retroviral vector, a recombinant herpes simplex virus vector or a recombinant vaccinia virus vector. Viral vector technology is well known in the art and described, for example, in Sambrook et al. (2001, Molecular Cloning: A Laboratory Manual, Cold Spring Harbor Laboratory, New York) and other handbooks of virology and molecular biology.

The nucleic acid constructs described herein can generally be obtained by PCR amplification. Specifically, primers can be designed according to the nucleotide sequence disclosed herein, especially the open reading frame sequence, and a commercially available cDNA library or a cDNA library prepared by a conventional method known to those skilled in the art can be used as a template, and related sequences were amplified. When the sequence is long, it is often necessary to carry out two or more PCR amplifications, and then assemble the amplified fragments in correct order. Alternatively, the nucleic acid constructs described herein can also be directly synthesized.

The invention also provides a host cell comprising the nucleic acid construct described in any of the embodiments herein. Host cells include both mammalian cells and various cells used in the production of mammalian cells, such as E. coli cells, for providing nucleic acid constructs or vectors as described herein. In certain embodiments, provided herein is a mammalian cell containing a nucleic acid construct or vector described herein, including but not limited to: T cells, B cells, CIK cells, LAK cells, NK cells, cytotoxic T lymphocytes (CTL), dendritic cells (DC), tumor infiltrating lymphocytes (TIL), macrophages, NK T cells, γδT cells, Jurkat cells, K562 cells, embryonic stem cells, tumor cells, HEK293 cells and CHO cells.

### pharmaceutical composition

The pharmaceutical composition of the present invention comprises the nucleic acid construct or cell described herein and pharmaceutically acceptable excipients. In the present invention, "pharmaceutically acceptable excipients" are pharmaceutically or food-acceptable carriers, solvents, suspending agents or excipients used to deliver the nucleic acid constructs or cells of the present invention to animals or humans. Herein, pharmaceutically acceptable excipients are nontoxic to recipients of the composition at the dosages and concentrations employed. Various types of carriers or excipients commonly used in the delivery of therapeutics in therapy known in the art may be included. Exemplary excipients can be liquid or solid and include, but are not limited to: pH adjusters, surfactants, carbohydrates, adjuvants, antioxidants, chelating agents, ionic strength enhancers, preservatives, carriers, glidants, sweeteners, dyes/colorants, flavor enhancers, wetting agents, dispersing agents, suspending agents, stabilizers, isotonic agents, solvents or emulsifiers. In some embodiments, pharmaceutically acceptable excipients may include one or more inactive ingredients, including but not limited to: stabilizers, preservatives, additives, adjuvants, sprays, compressed air or other suitable gases, Or other suitable inactive ingredients used in combination with medicinal compounds. More specifically, suitable adjuvants may be adjuvants commonly used in the art for administration of transposition systems or cells containing them. Examples of excipients include various lactoses, mannitols, oils such as corn oil, buffers such as PiggyBacS, saline, polyethylene glycol, glycerol, polypropylene glycol, dimethylsulfoxide, amides such as dimethylacetamide, proteins such as albumin, and detergents such as Tween 80, monosaccharides and oligopolysaccharides such as glucose, lactose, cyclodextrin and starch.

Other pharmaceutical compositions will be apparent to those skilled in the art, including formulations comprising the nucleic acid constructs or cells described herein in sustained or controlled release delivery formulations. Techniques for formulating a variety of other sustained or controlled delivery modes, such as liposomal vehicles, bioerodible microparticles or porous beads, and depot injections, are also known to those of skill in the art.

Pharmaceutical compositions for in vivo administration are usually provided in the form of sterile formulations. Sterilization is achieved by filtration through sterile filter membranes. When the composition is lyophilized, it can be sterilized using this method before or after lyophilization and reconstitution. Compositions for parenteral administration may be in lyophilized form or stored in solution. Parenteral compositions are usually presented in containers with sterile access pores, such as intravenous solution strips or vials with a hypodermic needle pierceable stopper.

Typically, the composition contains a therapeutically effective amount of an agent described herein. A therapeutically effective amount refers to a dose that can achieve treatment, prevention, alleviation and/or amelioration of a disease or condition in a subject. These effects can be realized by inserting exogenous genes with corresponding functions, and the exogenous genes with corresponding functions have functions corresponding to specific uses, such as therapeutic functions or induction functions. The therapeutically effective dose can be determined according to factors such as the patient's age, sex, disease and its severity, and other physical conditions of the patient. A therapeutically effective amount may be administered as a single dose, or may be administered in multiple doses in accordance with an effective treatment regimen. Herein, a subject or a patient generally refers to a mammal, especially a human. Exemplarily, the composition contains, for example, 0.001-50%, preferably 0.01-30%, more preferably 0.05-10% of the nucleic acid construct or cell described herein by weight.

The compositions described herein can be used in combination with other agents that have similar or corresponding functions to those performed by the exogenous genes. For example, it can be used in combination with an agent for treating the disease or condition treated by the exogenous gene. Dosages of other agents to be administered can be determined by those skilled in the art.

The dosage form of the pharmaceutical composition of the present invention can be varied, as long as the active ingredient can effectively reach the mammalian body, and can be made into the form of unit dosage. Dosage forms can be selected from, for example, gels, aerosols, tablets, capsules, pulvis, granules, syrups, solutions, suspensions, injections, powders, pills, controlled immediate releases, infusions, suspensions, etc. According to the types of diseases to be prevented and treated by the nucleic acid constructs or cells described herein, those skilled in the art can choose convenient dosage forms. From the standpoint of ease of preparation and storage, preferred compositions are solid compositions, especially tablets and solid-filled or liquid-filled capsules. The nucleic acid constructs or cells described herein, or compositions thereof, may also be stored in sterile devices suitable for injection or infusion. The nucleic acid constructs or cells described herein, or compositions thereof, may also be stored in suitable containers and placed in kits or drug packages.

### Method and Use

The inventors of the present invention found in research that the nucleic acid construct herein can efficiently realize genome integration of exogenous genes in a controllable manner. When the exogenous gene is integrated into the genome, it can effectively terminate the transcription and expression of PiggyBac transposase, and at the same time, it can function as an insulator of the exogenous gene expression cassette, reducing the impact of the integrated exogenous gene expression cassette on gene expression near the integration site. Therefore, the present invention relates to a method for integrating an exogenous gene or its expression cassette into the genome of a cell, comprising introducing the nucleic acid construct described in any embodiment herein containing the exogenous gene and optionally its promoter into the cells, and optionally incubating the cells under conditions in which the PiggyBac transposase integrates the exogenous gene or its expression cassette into the genome of the cell. The present invention also provides cells in which exogenous genes or its expression cassette is integrated in the genome obtained by the method described herein.

Methods for introducing nucleic acid constructs into cells are known in the art. Vectors can be readily introduced into host cells, eg, mammalian, bacterial, yeast or insect cells, by any method known in the art. For example, vectors can be transfected into host cells by physical, chemical or biological means. Exemplary physical or chemical methods include: calcium phosphate precipitation, lipofection, microinjection, particle bombardment, microinjection, biolistic transformation, electroporation, colloidal dispersion systems, macromolecular complexes, nanocapsules, micropheres, beads, lipid-based systems (including oil-in-water emulsions, micelles, mixed micelles, and liposomes). Biological methods for introducing nucleic acid constructs into host cells include viral-mediated transformation, particularly retroviral vectors. Other viral vectors can be derived from lentiviruses, poxviruses, herpes simplex virus I, adenoviruses, and adeno-associated viruses, etc. Insertion of selected nucleic acid sequences into vectors and packaging into retroviral particles can be performed using techniques known in the art. The recombinant virus can then be isolated and delivered to subject cells in vivo or ex vivo. Reagents for virus packaging are well known in the art, for example, conventional lentiviral vector systems include pRsv-REV, pMDlg-pRRE, pMD2G and objective interfering plasmids.

The present invention also provides the use of the nucleic acid construct or host cell described in any embodiment herein in the preparation of or as a medicament, reagent or tool for integrating an exogenous gene expression cassette into a target cell genome, or for gene therapy, cell therapy, stem cell induction or differentiation. In one or more embodiments, the target cells are mammalian cells, including but not limited to T cells, B cells, CIK cells, LAK cells, NK cells, cytotoxic T lymphocytes (CTL), dendritic cells (DC), tumor infiltrating lymphocytes (TIL), macrophages, NK T cells, γδT cells, Jurkat cells, K562 cells, embryonic stem cells, tumor cells, HEK293 cells and CHO cells.

### Exemplary embodiments

Item 1,a nucleic acid construct, which comprises or consists of the following elements: a transposon 3' terminal repeat sequence, a first polyA sequence, an insulator sequence with transcription termination function, a transposon 5' terminal repeat sequence,
preferably, the nucleic acid construct further comprises one or more elements selected from the following: a transposase coding sequence, a promoter controlling the expression of the transposase, a multiple cloning insertion site, an enhancer, a 5'UTR, a second polyA sequence and an exogenous gene of interest.
preferably, any one or more of the transposase coding sequence, the promoter controlling the expression of the transposase, the 5'UTR and the second polyA sequence are outside of the region between the transposon 3' terminal repeat and the transposon 5' terminal repeat.

Item2. The nucleic acid construct according to item1, which comprises the following elements: a transposon 3' terminal repeat sequence,a first polyA sequence, an insulator sequence with transcription termination function, a transposon 5' terminal repeat sequence, a transposase encoding sequence and a promoter controlling the expression of the transposase,
preferably, the nucleic acid construct further comprises one or more elements selected from the group consisting of: a multiple cloning insertion site, an enhancer, a 5'UTR, second polyA sequence and an exogenous gene of interest.

Item 3. The nucleic acid construct according to item 2, wherein,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a transposase coding sequence and a promoter for controlling transposase expression,
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a transposase coding sequence and a promoter for controlling transposase expression,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a transposase coding sequence, a 5'UTR and a promoter for controlling transposase expression,
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a transposase coding sequence, a 5'UTR and a promoter for controlling transposase expression,
the nucleic acid construct comprises sequentially: a transposon 3' terminal repeat sequence, a first polyA sequence, an insulator sequence with transcription termination function, a transposon 5' terminal repeat sequence, a promoter for controlling transposase expression, a transposase coding sequence and a second polyA sequence,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a promoter for controlling transposase expression, a transposase coding sequence and a second polyA sequence,
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a promoter for controlling transposase expression, a transposase coding sequence and a second polyA sequence,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a promoter for controlling transposase expression, a 5'UTR, a transposase coding sequence and a second polyA sequence, or
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a promoter for controlling transposase expression, a 5'UTR, a transposase coding sequence and a second polyA sequence,
the nucleic acid construct comprises sequentially: a transposon 3' terminal repeat sequence, an insulator sequence with transcription termination function, a multiple cloning insertion site, a first polyA sequence, a transposon 5' terminal repeat sequence, a promoter for controlling transposase expression, a 5'UTR, a transposase coding sequence and a second polyA sequence,
the nucleic acid construct comprises sequentially: a transposon 3' terminal repeat sequence, an insulator sequence with transcription termination function, a multiple cloning insertion site, a first polyA sequence, en enhancer, a transposon 5' terminal repeat sequence, a promoter for controlling transposase expression, a 5'UTR, a transposase coding sequence and a second polyA sequence , or
the nucleic acid construct comprises sequentially: a transposon 3' terminal repeat sequence, an enhancer, an insulator sequence with transcription termination function, a multiple cloning insertion site, a first polyA sequence, a transposon 5' terminal repeat sequence, a promoter for controlling transposase expression, a 5'UTR, a transposase coding sequence and a second polyA sequence.

Item 4. The nucleic acid construct according to any one of items 1-3, wherein the nucleic acid construct has one or more characteristics selected from the group consisting of:
the orientation of the expression cassette of the transposase is the same as or opposite to the orientation of the expression cassette of the exogenous gene,
the orientation of the expression cassette of the transposase is the same as or opposite to the orientation of the sequence between the transposon 3' terminal repeat sequence and the transposon 5' terminal repeat sequence,
the position of the transposon 5' terminal repeat sequence and the position of the transposon 3' terminal repeat sequence can be interchanged,
the transposon 3' terminal repeat sequence is the PiggyBac transposon 3' terminal repeat sequence,
the transposon 5' terminal repeat sequence is the PiggyBac transposon 5' terminal repeat sequence,
the enhancer is selected from the group consisting of: CMV enhancer sequence, SV40 enhancer, human epsilon globin 5' HS2 enhancer, chicken β globin gene 5' HS4 enhancer,
the transposase is PiggyBac transposase,
the 5'UTR is selected from the group consisting of: the 5'UTR of C3 gene, ORM1 gene, HPX gene, FGA gene, AGXT gene, ASL gene, APOA2 gene, ALB gene,
the promoter is selected from the group consisting of: CMV promoter, miniCMV promoter, CMV53 promoter, miniSV40 promoter, miniTK promoter, MLP promoter, pJB42CAT5 promoter, YB_TATA promoter, EF1α promoter, SV40 promoter, UbiquitinB promoter, CAG promoter, HSP70 promoter, PGK-1 promoter, β-actin promoter, TK promoter and GRP78 promoter,
the transposase coding sequence contains or is operably linked to a single copy or multiple copies of a nuclear localization signal coding sequence.

Item 5. The nucleic acid construct according to any one of items 1-3, wherein the nucleic acid construct has one or more characteristics selected from the the group consisting of:
the nucleotide sequence of the transposon 3' terminal repeat sequence is as shown in SEQ ID NO: 1,
the nucleotide sequence of the transposon 5' terminal repeat sequence is as shown in SEQ ID NO: 6,
the sequence of the multiple cloning insertion site is as shown in SEQ ID NO: 2,
the first polyA sequence is as shown in SEQ ID NO: 3, 13 or 16,
the second polyA sequence is as shown in SEQ ID NO: 3, 13 or 16,
the enhancer sequence is as shown in any one of SEQ ID NO:4, 26-28.
the insulator sequence is as shown in SEQ ID NO: 5 or 15,
the amino acid sequence of the PiggyBac transposase is as shown in SEQ ID NO:36; preferably, the coding sequence of the PiggyBac transposase is as shown in SEQ ID NO:7,
the 5'UTR sequence is as shown in any one of SEQ ID NO:8, 17-24.
the sequence of the promoter is as shown in any one of SEQ ID NO:9, SEQ ID NO:37-42;
the nuclear localization signal is a c-myc nuclear localization signal; preferably, the nuclear localization signal has the sequence shown in SEQ ID NO:35.

Item 6. The nucleic acid construct according to any one of items 1-3, characterized in that,
The nucleic acid construct comprises a sequence shown in SEQ ID NO: 10 or 14, or
the nucleic acid construct is a recombinant vector, preferably, the nucleic acid construct is a recombinant cloning vector or a recombinant expression vector.

Item 7. A host cell comprising
(1) the nucleic acid construct described in any one of items 1-6, and/or
(2) the sequence between the transposon 3' terminal repeat sequence and the transposon 5' terminal repeat sequence of the nucleic acid construct described in any one of items 1-6,
   preferably, the host cell is a mammalian cell,
   more preferably, the host cells are selected from the group consisting of: T cells, Jurkat cells, K562 cells, embryonic stem cells, tumor cells, HEK293 cells and CHO cells.

Item 8. A pharmaceutical composition, comprising the nucleic acid construct described in any one of Items 1-6 or the host cell described in Item 8 and pharmaceutically acceptable excipients.

Item 9. Use of the nucleic acid construct described in any one of Items 1-6 or the host cell described in Item 7 in the preparation of or as a medicament, reagent or tool, wherein, the medicament, reagent or tools are used for the integration of exogenous gene expression cassettes into the genome of target cells, or for gene therapy, cell therapy, stem cell induction or differentiation,
preferably, the target cells are mammalian cells,
more preferably, the target cells are selected from the group consisting of:immune cells, Jurkat cells, K562 cells, embryonic stem cells, tumor cells, HEK293 cells and CHO cells.
further more preferably, the immune cells are selected from any one or more of the group consisting of: T cells, B cells, CIK cells, LAK cells, NK cells, cytotoxic T lymphocytes (CTL), dendritic cells (DC), tumor infiltrating lymphocytes (TIL), macrophages, NK T cells, and γδT cells.

Item 10. A method for integrating an exogenous gene or its expression cassette into the genome of a cell, comprising introducing into said cells the nucleic acid construct described in any one of items 1-6 containing the exogenous gene and optionally its promoter, and optionally incubating said cells under conditions in which a transposase integrates an exogenous gene or its expression cassette into the genome of the cell, said exogenous gene and optionally its promoter are located in the multiple cloning insertion site of said nucleic acid construct,
preferably, the transposase is PiggyBac transposase.

Advantages of the present invention:
1) The transposon vector system of the present invention has multiple regulatory elements to regulate the expression of PiggyBac and the integration of exogenous genes mediated thereby at multiple levels. The promoter used herein to drive the expression of the PiggyBac transposase has reduced expression intensity and shortened DNA length. Meanwhile, enhancer elements are introduced to transiently enhance the expression of the transposase in the complete form of plasmid, enabling the cutting and integration functions of PiggyBac transposase; when the exogenous gene expression cassette is cut from the complete plasmid, the enhancer's enhancing effect of the promoter is disabled, thus achieving efficient on-off switching of PiggyBac transposase, so that the level of transposase can be reduced in a short period of time after reaching its peak, thus mediating the integration of exogenous genes with greatly reduced cytotoxicity;
2) A 5'UTR sequence is added downstream of the promoter, which transiently increases the expression intensity of the transposase through coordination with the enhancer element, so that the expression intensity of the PiggyBac enzyme is high enough to improve the overall integration efficiency. As shown in the following examples, the integration efficiency of the transposon vector system of the present invention is significantly improved;
3) The PiggyBac expression cassette adopts an insulator sequence with transcription termination function, which can effectively terminate transcription and express the transposase gene before the integration of the exogenous gene occurs; after the exogenous gene is integrated into the genome, the absence of the insulator sequence with transcription termination function can effectively terminates transcriptional expression of PiggyBac transposase. In the meantime, after the insulator sequence with transcription termination function is integrated into the genome together with the exogenous gene expression cassette, the insulator can also block the influence of the expression of the exogenous gene on its adjacent regions of the genome, reducing the effects of the integrated exogenous gene expression cassette on the expression of genes residing in the proximity of the integration site;
4) The transposon vector system of the present invention has a limited number of genomic and intragenic insertion sites, which produces minimal impact on genome stability;
5) Minor effects on gene expression profile at mRNA level.

The embodiments involved in the present invention will be described in detail below with examples. Those skilled in the art will understand that the following examples are only used to illustrate the present invention, and should not be considered as limiting the scope of the present invention. Where techniques or conditions in the examples are not specified, experimental operation shall follow the techniques or conditions described in the literature in this field (for example, refer to J.Sambrook et al., "Molecular Cloning Experiment Guide" translated by Huang Peitang et al., third edition, Science Press) or follow the manufacturer's instructions. The used reagents or instruments, the manufacturers of which were not specifically indicated, are all commercially available conventional products. All cell lines used in the following examples were purchased from ATCC.

### Example

### Example 1, The Construction of Vectors

The PiggyBac transposon 3' terminal repeat sequence (3'ITR) (SEQ ID NO: 1), multiple cloning site (SEQ ID NO: 2), bGH polyA signal sequence (SEQ ID NO: 3), enhancer motif sequence (SEQ ID NO:4), insulator sequence with transcription termination function (C2 transcription pause site, SEQ ID NO:5), the reverse complement sequence of PiggyBac transposon 5' terminal repeat sequence (5'ITR) (SEQ ID NO:6), the reverse complement sequence of the PiggyBac transposase coding sequence (SEQ ID NO:7), the reverse complement sequence of the 5'UTR sequence (SEQ ID NO:8), and the reverse complement sequence of miniCMV promoter sequence (SEQ ID NO:9) were assembled sequentially into a long fragment (SEQ ID NO: 10), and was synthesized by Shanghai Generay Biotechnology Co., Ltd..The long fragment was added with AgeI and AscI on both ends and was cloned into pUC57 (purchased from Shanghai Generay Biotech Co., Ltd.), The obtained vector was named pKB20. A schematic diagram of the plasmid is as shown in Figure 1A.

pKB20-EGFP: the EF1A promoter sequence (SEQ ID NO:11) with NFAT motif was inserted between the XbaI and EcoRI sites in the multiple cloning site of pKB20, and the EGFP coding sequence was inserted between the EcoRI and SalI sites (SEQ ID NO:12). The obtained vector was named pKB20-EGFP. the EF1A promoter sequence with NFAT motif and the EGFP coding sequence were synthesized by Shanghai Generay Biotechnology Co., Ltd.

pKB205: the following elements were assembled into a long fragment (SEQ ID NO:14) sequentially: PiggyBac transposon 3' terminal repeat sequence (3'ITR) (SEQ ID NO: 1), multiple cloning site (SEQ ID NO: 2), bGH polyA signal sequence (SEQ ID NO: 3), enhancer motif sequence (SEQ ID NO:4), insulator sequence with transcription termination function (C2 transcription pause site, SEQ ID NO:5), PiggyBac transposon 5' terminal repeat sequence (5'ITR) (SEQ ID NO:6), miniCMV promoter sequence (SEQ ID NO:9), 5'UTR sequence (SEQ ID NO:8), PiggyBac transposase coding sequence (SEQ ID NO:7) and SV40 polyA signal sequence (SEQ ID NO:13).The long fragment was synthesized by Shanghai Generay Biotechnology Co., Ltd., with AgeI and AscI restriction sites added at both ends, and cloned into pUC57 (purchased From Shanghai Generay Biotechnology Co., Ltd). The obtained vector was named pKB205. A schematic diagram of the plasmid is as shown in Figure 1B.

pKB205-EGFP: the EF1A promoter sequence (SEQ ID NO:11) with NFAT motif was inserted between the XbaI and EcoRI sites in the multiple cloning site of pKB205, and the EGFP coding sequence was inserted between the EcoRI and SalI sites ( SEQ ID NO:12). The obtained vector was named pKB205-EGFP. The EF1A promoter sequence with NFAT motif and EGFP coding sequence was synthesized by Shanghai Generay Biotechnology Co., Ltd.

The following plasmids were obtained by the same method:
pKB201-EGFP: obtained by removing the enhancer motif sequence from pKB20-EGFP sequence;
pKB201-EGFP: obtained by removing the 5'UTR sequencefrom pKB20-EGFP sequence ;
pKB2003-EGFP: obtained by removing the enhancer motif sequence and 5'UTR sequence from pKB20-EGFP sequence;
pKC20-EGFP: obtained by removing the miniCMV promoter sequence, 5'UTR sequence, and PiggyBac transposase coding sequence containing nuclear localization signal from pKB20-EGFP sequence;
pK201-PB: obtained by removing the 5'UTR, 5'ITR, enhancer motif, bGH polyA signal sequence, multiple cloning site and 3'ITR from the pKB20 sequence, retaining only the miniCMV, PiggyBac transposase coding sequence with nuclear localization signal and insulator sequence with transcription termination function;
pKB20I1-EGFP: obtained by replacing the insulator sequence with transcription termination function in pKB20-EGFP with the human α2globin gene transcription pause site (SEQ ID NO: 15);
pKB20A1-EGFP: obtained by replacing the bGH polyA in pKB20-EGFP with SEQ ID NO: 16;
pKB20A2-EGFP: obtained by replacing the bGH polyA in pKB20-EGFP with the SV40 polyA signal sequence (SEQ ID NO: 13);
pKB20U1-EGFP: obtained by replacing the 5'UTR sequence in pKB20-EGFP with the 5'UTR of the C3 gene (SEQ ID NO: 17);
pKB20U2-EGFP: obtained by replacing the 5'UTR sequence in pKB20-EGFP with the 5'UTR of the ORM1 gene (SEQ ID NO: 18);
pKB20U3-EGFP: obtained by replacing the 5'UTR sequence in pKB20-EGFP with the 5'UTR of the HPX gene (SEQ ID NO: 19);
pKB20U4-EGFP: obtained by replacing the 5'UTR sequence in pKB20-EGFP with the 5'UTR of the FGA gene (SEQ ID NO:20);
pKB20U5-EGFP: obtained by replacing the 5'UTR sequence in pKB20-EGFP with the 5'UTR of the AGXT gene (SEQ ID NO:21);
pKB20U6-EGFP: obtained by replacing the 5'UTR sequence in pKB20-EGFP with the 5'UTR of the ASL gene (SEQ ID NO:22);
pKB20U7-EGFP: obtained by replacing the 5'UTR sequence in pKB20-EGFP with the 5'UTR of the APOA2 gene (SEQ ID NO:23);
pKB20U8-EGFP: obtained by replacing the 5'UTR sequence in pKB20-EGFP with the 5'UTR of the ALB gene (SEQ ID NO:24);
pKB20E1-EGFP: obtained by replacing the enhancer motif sequence in pKB20-EGFP with the CMV enhancer sequence (SEQ ID NO:25);
pKB20E2-EGFP: obtained by replacing the enhancer motif sequence in pKB20-EGFP with the SV40 enhancer sequence (SEQ ID NO:26);
pKB20E3-EGFP: obtained by replacing the enhancer motif sequence in pKB20-EGFP with the human ε-globin 5'HS2 enhancer sequence (SEQ ID NO:27);
pKB20E4-EGFP: obtained by replacing the enhancer motif sequence in pKB20-EGFP with the chicken β-globin gene 5'HS4 enhancer sequence (SEQ ID NO:28);
pKB20P1-EGFP: obtained by replacing the miniCMV sequence in pKB20-EGFP with the CMV53 promoter (SEQ ID NO:37);
pKB20P2-EGFP: obtained by replacing the miniCMV sequence in pKB20-EGFP with the miniSV40 promoter (SEQ ID NO:38);
pKB20P3-EGFP: obtained by replacing the miniCMV sequence in pKB20-EGFP with the miniTK promoter (SEQ ID NO:39);
pKB20P4-EGFP: obtained by replacing the miniCMV sequence in pKB20-EGFP with the MLP promoter (SEQ ID NO:40);
pKB20P5-EGFP: obtained by replacing the miniCMV sequence in pKB20-EGFP with the pJB42CAT5 promoter (SEQ ID NO:41);
pKB20P6-EGFP: obtained by replacing the miniCMV sequence in pKB20-EGFP with YB_TATA (SEQ ID NO:42);

### Example 2, the integration of pKB vector containing EGFP expression cassette in Jurkat cells

5×10⁶ vigorously growing low-passage Jurkat cells were prepared, and 5 µg of pKB20-EGFP, pKB201-EGFP, pKB202-EGFP, and pKC20-EGFP were transfected into the nucleus through the Lonza2b-Nucleofector instrument (according to the manufacturer's instructions), and the cells were placed in 37 °C, 5% CO₂ incubator. After the cells were confluent, they were passaged at a ratio of 1:10 for further culture. On Day 7, 10 and 14 post-transfection, fluorescence imaging was performed; on day 7, 10 and 14 (after 3 passages) post-transfection, flow cytometry assay was used to detect the change in the proportion of EGFP-positive cells, with non-transfected Jurkat cells acting as control. Cell fluorescence intensity was monitored by fluorescence microscope throughout the whole process of electrotransfection. Viable cell counts were performed on day 5, 7, 10 and 14 post-transfection to observe the effects of electrotransfection with pKB vector on the proliferation of Jurkat cells.

Cells were diluted and passaged at a ratio of 1:10, and the non-integrated plasmid were lost rapidly with cell division. After 3 passages (about 13 days), the green fluorescence-positive cells can be considered to have been stably integrated with the green fluorescent protein expression cassette. The efficiency of integration can be determined by measuring the proportion of green fluorescence-positive cells by flow cytometry.

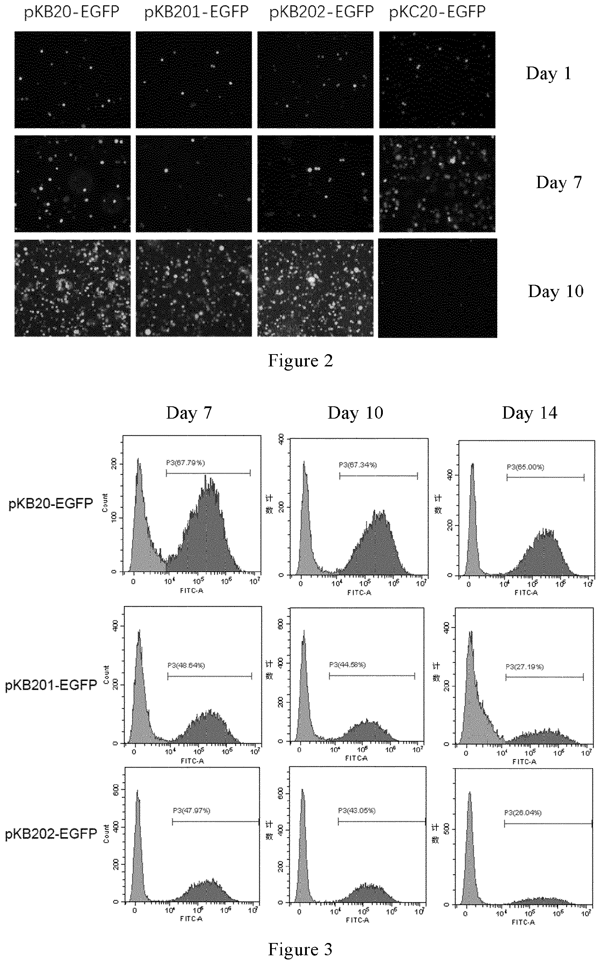

The results are shown in Figure 2-4. Figure 2 shows that on day 10 after electrotransfection, a large number of cells with high fluorescence intensity can still be seen in the Jurkat cells transfected with pKB20-EGFP, pKB201-EGFP, and pKB202-EGFP.. In the jurkat cells electrotransfected with pKC20-EGFP lacking the PiggyBac transposase expression cassette almost no fluorescence could be observed. This shows that the vector with the PiggyBac transposase expression cassette has successfully integrated EGFP into the genome of Jurkat cells. Vectors that do not include the PiggyBac transposase expression cassette cannot effectively mediate the integration of the exogenous gene EGFP.

Figure 3 shows the flow cytometry assay results of Jurkat cells electrotransfected with pKB20-EGFP, pKB201-EGFP and pKB202-EGFP, respectively on day 7 after electrotransfection. The results showed that the percentage of positive cells in Jurkat cells electrotransfected with pKB20-EGFP was as high as 67% both on day 7 and day 10, and the positive proportion remained at a level as high as 65% on day 14 after three passages. The positive proportion of cells electrotransfected with pKB201-EGFP and pKB202-EGFP was about 48% on the 7th day, and exceeded 27% and 26% on day 14 after passaging three times.

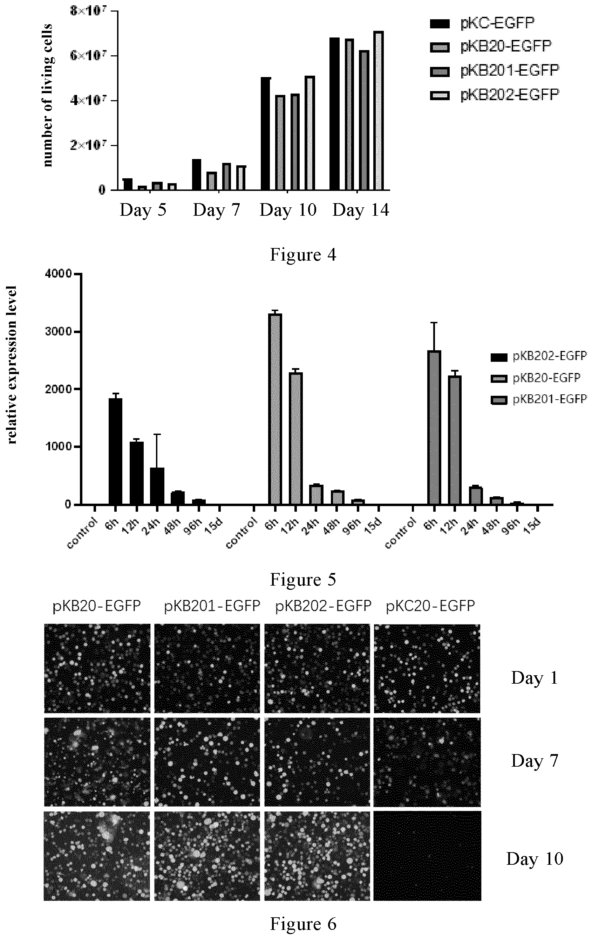

Figure 4 shows that compared with the Jurkat cells electrotransfected with pKC20-EGFP that do not contain the PB transposase expression cassette, there was no significant difference in the number of viable cells on days 5, 7, 10, and 14 after electrotransfection in Jurkat cells transfected with pKB20-EGFP, pKB201-EGFP and pKB202-EGFP which contain PB transposase expression cassette. This shows that the introduction of the PB transposase expression cassette does not affect the proliferation of Jurkat cells.

The above results show that pKB20-EGFP has a very high integration rate and positive expression rate of exogenous genes after electrotransfection into Jurkat cells. pKB201-EGFP and pKB202-EGFP also have high integration rates and positive expression rates of exogenous genes after electrotransfection into Jurkat cells, but lower than that of pKB20-EGFP. And the integration of the pKB series vectors basically has no effect on cell proliferation.

### Example 3, PB transposase time-course expression curve after pKB vector electrotransfection into Jurkat cells

5×10⁶ vigorously growing low-passage Jurkat cells were prepared, and 5 µg of pKB20-EGFP, pKB201-EGFP, and pKB202-EGFP were transfected into the nucleus with the Lonza2b-Nucleofector instrument (according to the maufacturer's instructions). Cells were incubated at 37 °C, 5% CO₂. On the 6th, 12th, 24th, 48th, 96th hour and 15th day after electrotransfection, cells were collected and RNA was extracted, and the expression level of PB transposase was quantitatively measured by RT-PCR with β-actin acting as the internal reference. The result is shown in Figure 5.

Figure 5 shows that the expression levels of PB transposase in Jurkat cells electrotransfected with pKB20-EGFP, pKB201-EGFP and pKB202-EGFP all reached their peak at the 6^{th} hour, and then began to decrease significantly. The expression level of PB transposase in the electrotransfected pKB20-EGFP and pKB201-EGFP cells at the 6^{th} hour peak was significantly higher than that of the cells electrotransfected with pKB202-EGFP. The expression level of PB transposase in the cells electrotransfected with pKB20-EGFP and pKB201-EGFP decreased drastically at the 24^{th} hour. The expression level of PB transposase in all cells dropped to a very low level at the 96th hour, and could not be detected by the 15th day.

### Example 4, the integration of pKB vector containing EGFP expression cassette in K562 cells

5×10⁶ vigorously growing low-passage K562 cells were prepared, and 5µg of pKB20-EGFP, pKB201-EGFP, pKB202-EGFP, and pKC20-EGFP each was tranfected into the nucleus through the Lonza2b-Nucleofector instrument (according to the manufacturer's instructions), and cells were cultured in a 37 °C, 5% CO₂ incubator. After the cells were confluent, they were subcultured at a ratio of 1:10. On Day 1, 7 and 10 post-transfection, fluorescence microscopic imaging was performed; on day 10 and 14 (after 3 passages) after the electrotransfection, flow cytometry assay was performed to detect the change in the proportion of EGFP-positive cells, with non-transfected K562 cells acting as control. Cell fluorescence intensity was monitored by fluorescence microscope throughout the whole process of electrotransfection. Viable cell counts were performed on day 5, 7, 10 and 14 pos-transfection to observe the effects of electrotransfection with pKB vector on the proliferation of K562 cells.

Diluting and passaging at a ratio of 1:10, the non-integrated plasmid is lost rapidly with cell division. After 3 passages (about 13 days), the green fluorescence-positive cells can be considered to have been stably integrated with the green fluorescent protein expression cassette. The efficiency of integration can be determined by measuring the proportion of green fluorescence-positive cells by flow cytometry.

The results are shown in Figure 6-9. Figure 6 shows that on day 10 after electrotransfection, a large number of cells with high fluorescence intensity can still be seen in the K562 cells transfected with pKB20-EGFP, pKB201-EGFP, and pKB202-EGFP. In K562 cells electrotransfected with pKC20-EGFP lacking the PiggyBac transposase expression cassette almost no cells with high fluorescence intensity could be observed. This shows that the vector with the PiggyBac transposase expression cassette has successfully integrated EGFP into the genome of K562 cells. Vectors that does not include the PiggyBac transposase expression cassette cannot effectively mediate the integration of the exogenous gene EGFP.

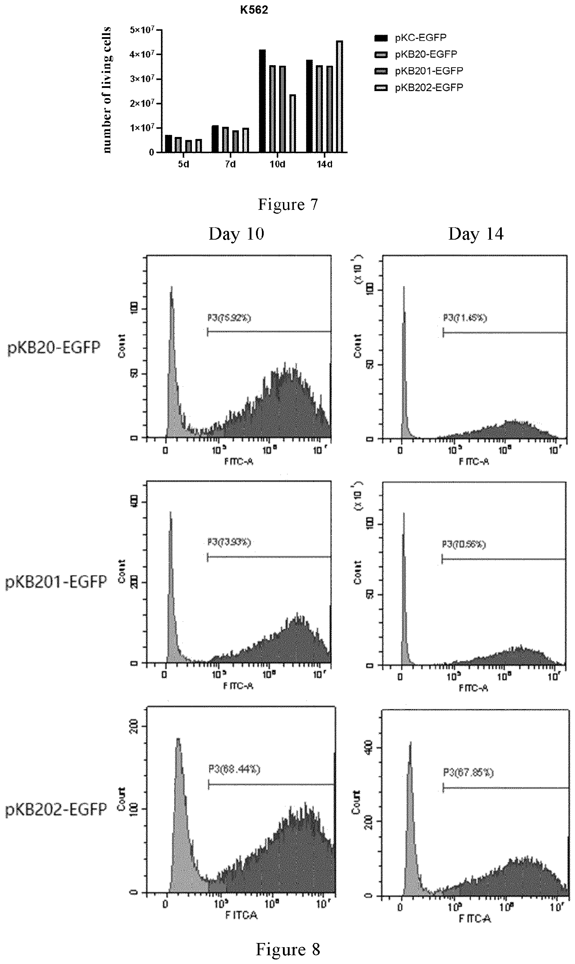

Figure 7 shows that cells electrotransfected with plasmids pKB20-EGFP, pKB201-EGFP and pKB202-EGFP containing the PB transposase expression cassette, when compared with the cells electrotransfected with pKC20-EGFP that does not contain the PB transposase expression cassette, shows no significant difference in the number of viable cells on day 5, 7, 10, and 14 post-transfection. This shows that the introduction of the PB transposase expression cassette does not affect the proliferation of K562 cells.

Figure 8 shows the flow cytometry assay results of the cells transfected with pKB20-EGFP, pKB201-EGFP and pKB202-EGFP on day 10 and 14 post-tranfection, respectively. The results showed that the fluorescence-positive proportions of cells electrotransfected with pKB20-EGFP and pKB201-EGFP exceeded 75% and 73%, respectively, on the day 10, and the fluorescence-positive proportions remained at 70% on day 14 post-transfection (cultured for more than 3 passages), showing a very high integration efficiency. The fluorescence-positive proportion of the cells electrotransfected with pKB202-EGFP was also close to 70% on day 10 post-transfection, and the fluorescence-positive proportion remained at a level close to 70% on day 14 post-transfection (cultured for more than 3 passages).

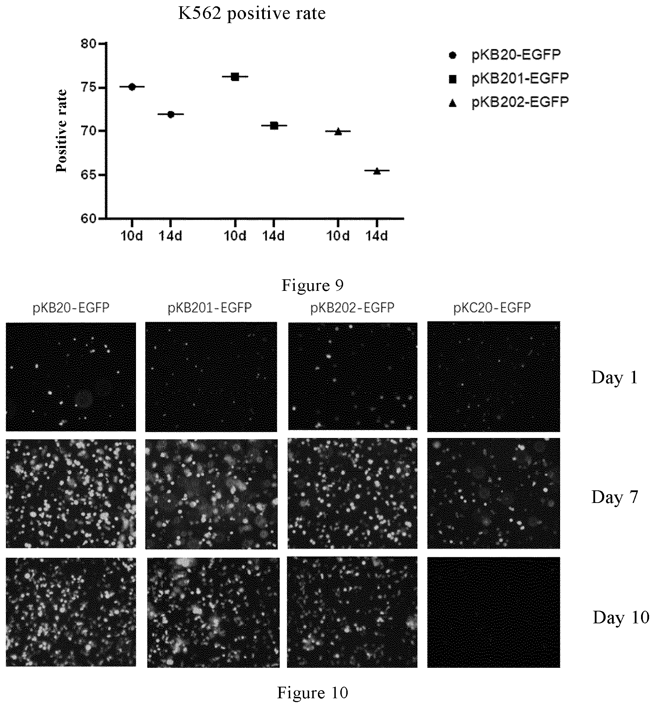

Figure 9 shows that from day10 to day14 post-transfection, the fluorescence-positive proportion of K562 cells transfected with pKB20-EGFP and pKB201-EGFP vectors did not variate much, both being above 70% and close to 75%; The fluorescence-positive proportion of cells did not decrease significantly between day10 and day14 day post-transfection, which was slightly lower than 70%.

The above results indicated that pKB20-EGFP, pKB201-EGFP and pKB202-EGFP all had very high integration rate and positive expression rate of exogenous gene after electrotransfection into K562 cells.

### Example 5, the integration of pKB vector containing EGFP expression cassette in primary T cells

Four groups of freshly isolated peripheral blood mononuclear cells (PBMC) were prepared, 5×10⁶ cells in each group, and 5 µg of pKB20-EGFP, pKB201-EGFP, pKB202-EGFP and pKC20 - EGFP each was electrotransfected into the cell nucleus (according to the manufacturer's instruction), and the cells were placed in AIM-V medium and cultured in a 37° C, 5% CO₂ incubator post-tranfection. After 6 hours, cells were transferred to a 6-well plate containing 30ng/mL anti-CD3 antibody and 3000IU/mL IL-2 (purchased from Novoprotein), and cultured in a 37°C, 5% CO₂ incubator. Upon reaching confluency, cells were diluted and passaged at a ratio of 1:10, and fluorescent microscopic imaging was performed on the cells electrotransfected with pKB20-EGFP, pKB201-EGFP, pKB202-EGFP, and pKC20-EGFP on the day1, 7 and 10 post-transfection, respectively. Flow cytometric assay was conducted on the cells electrotransfected with pKB20-EGFP, pKB201-EGFP and pKB202-EGFP on day7, 10 and 14 post-transfection, and the results are shown in Figures 10-11.

Figure 10 shows that on day10 post-transfection, a large number of cells with high fluorescence intensity can still be seen in the T cells transfected with pKB20-EGFP, pKB201-EGFP, and pKB202-EGFP. In T cells electrotransfected with pKC20-EGFP lacking the PiggyBac transposase expression cassette cassette could cells with high fluorescence intensity hardly been observed . This shows that the vector with the PiggyBac transposase expression cassette has successfully integrated EGFP into the genome of T cells. Vectors that do not include the PiggyBac transposase expression cassette cannot effectively mediate the integration of the exogenous gene EGFP.

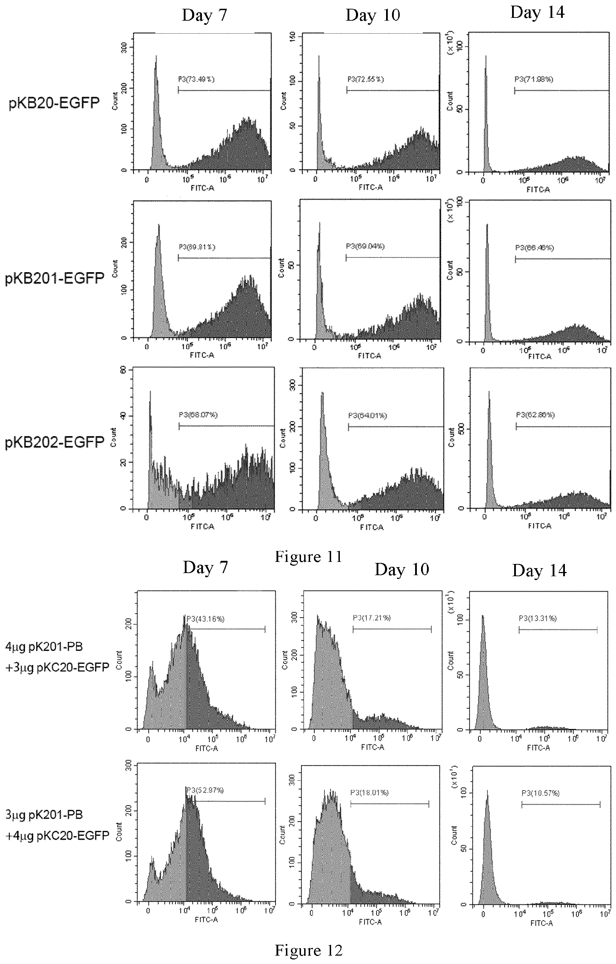

Figure 11 shows the flow cytometry assay results of T cells electrotransfected with pKB20-EGFP, pKB201-EGFP and pKB202-EGFP from day7 to 14 post-transfection. The results showed that the positive proportion of T cells transfected with pKB20-EGFP was close to 74% on day7, and remained close to 72% on day14 post-transfection (cultured for 3passages). The positive proportions of T cells electrotransfected with pKB201-EGFP and pKB202-EGFP were also as high as nearly 70% on day7 post-transfection, and the positive proportions remained above 66% and 62%, respectively on day14 post-transfection (cultured for 3 passages) The above results indicated that pKB20-EGFP, pKB201-EGFP and pKB202-EGFP all have very high integration rates and positive expression rates of exogenous genes after electrotransfection of primary T cells.

### Example 6, Detection of integration efficiency after electrotransfection of Jurkat cells with dual-plasmid PB transposition system

1×10⁷ vigorously growing Jurkat cells with low passage number were prepared and divided into two groups, A and B, with 5×10⁶ cells in each group. 4 µg of pK201-PB + 3 µg of pKC20-EGFP mixture and 3 µg of pK201-PB + 4 µg of pKC20-EGFP mixture, were prepared, respectively, and were electrotransfected into the nucleus of cells in groups A and B via the Lonza2b-Nucleofector instrument (according to manufacturer's instruction), before they were cultured in a 37 ° C, 5% CO₂ incubator. When the cells reached confluency, they were subcultured at a ratio of 1:10. , Fluorescent microscopic imaging was conducted on the day7, 10, 14 post-transfection, and the proportion of EGFP-positive cells was measured by flow cytometry on day7, 10, and 14 (after 3 passages of culture) post-transfection, with non-transfected Jurkat serving as control.

The results are shown in Figure 12. The results showed that the positive proportion of Jurkat cells electrotransfected with 4 µg of pK201-PB+3 µg of pKC20-EGFP exceeded 43% on day7 post-transfection, and decreased to 13.31% on day14 post-transfection (after 3 passages of culture). Similarly, the positive proportion of Jurkat cells electrotransfected with 3 µg of pK201-PB+4 µg of pKC20-EGFP exceeded 52% on day 7 post-transfection, and decreased to 10.57% on day 14 post-transfection (after 3 passages of culture).

The above results indicated that the integration efficiency of the dual-vector transposition system expressing PB transposase and exogenous gene separately in Jurkat cells was much lower than that of the aforementioned single-vector pKB system.

### Example 7, Detection of integration efficiency after electrotransfection of primary T cells with dual-plasmid PB transposition system

5×10⁶ freshly isolated peripheral blood mononuclear cells (PBMC) and 4 µg of pK201-PB+3 µg of pKC20-EGFP mixture were prepared, and Lonza Nucleofector-2b electroporator was used to electrotransfect the vector into the cell nucleus (according to the manufacturer's instruction). After electrotransfection, the cells were placed in AIM-V medium and cultured in a 37°C, 5% CO₂ incubator. After 6 hours, the cells were transferred to a 6-well plate containing 30ng/mL anti-CD3 antibody and 3000IU/mL IL-2 (purchased from Novoprotein), and cultured in a 37°C, 5% CO₂ incubator. Upon reaching confluency, the cells were diluted and passaged at a ratio of 1:10, and were submitted to flow cytometry assay on day7 and 14 post-transfection.

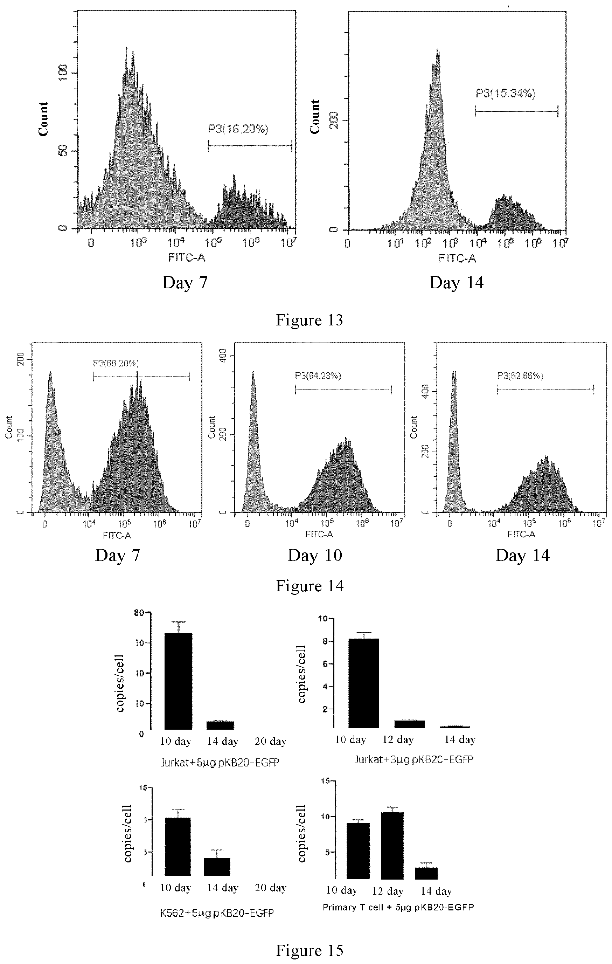

The results are shown in Figure 13. The results showed that the positive proportion of the primary cells electrotransfected with 4 µg of pK201-PB+3 µg of pKC20-EGFP was 16.20% on day7 post-transfection, and was reduced to 15.34% on day14 post-transfection (after 3 passages of culture).

The above results indicated that the integration efficiency of the dual-vector transposition system expressing PB transposase and exogenous genes separately in primary T cells was significantly lower than that of the aforementioned single-vector pKB system.

### Example 8, Detection of integration efficiency after electrotransfection of Jurkat cells with pKB vector with reduced amount of plasmid

5×10⁶ vigorously growing low-passage Jurkat cells and 3 µg of pKB20-EGFP plasmid were prepared, and electrotransfected into the nucleus via Lonza2b-Nucleofector instrument (according to manufacturer's instruction), and cultured in a 37°C, 5% CO₂ incubator. Upon reaching confluency, cells were subcultured at a ratio of 1:10. The proportion of EGFP-positive cells was measured by flow cytometry on day7, 10 and 14 post-transfection (after 3 passages of culture), and non-transfected Jurkat cells were used as control.

The results are shown in Figure 14. The results showed that the positive proportion of Jurkat cells electrotransfected with 3 µg pKB20-EGFP plasmid exceeded 66% on day7post-transfection, and remained close to 63% on day14 post-transfection (after 3 passages of culture), which is close to the positive portion of cells transfected with 6 µg of pKB20-EGFP plasmid on day 14 post-transfection in Example 2.

The above results show that when the amount of pKB20-EGFP plasmid used for electrotransfection is reduced by half on the basis of Example 2, the integration efficiency of the pKB vector system in cells remains basically unchanged, indicating that the pKB vector system of the present invention can achieve equivalent integration efficiency with reduced amount of DNA, which is greatly conducive to the reduction of amount of DNA used in electrotransfection, thus reducing the DNA-derived T cell toxicity as well as residual cellular plasmid DNA

### Example 9, Detection of intracellular pKB vector residual plasmid copy number

According to the method of Examples 2 and 4, 5 µg of pKB20-EGFP was electrotransfected into Jurkat and K562 cells, respectively, and the cells were harvested on day10, 14 and 20 post-transfection; according to the method of Example 8, 3 µg of pKB20-EGFP was electrotransfected into Jurkat cells per manufacturer's instructions, and the cells were harvested on day 10, 12 and 14 post-transfection, respectively. All the operations were repeated 3 times. The Taqman fluorescence probe quantitative PCR method was used to detect the amount of residual plasmid at different time points for all the above harvested cells containing the PB transposase expression cassette:
1) Use the pKB20-EGFP plasmid containing the PB transposase expression cassette as the standard, and use the plasmid containing the internal reference gene Actin as the internal reference standard to prepare 10-fold serially diluted standards;
2) Extract the total DNA of the cells at the specified number of days after electrotransfection of pKB20-EGFP as the sample for detecting PB transposase gene and Actin gene;
3) Prepare the PCR reaction system, and amplify PB transposase gene and internal reference Actin gene, respectively, for the samples to be tested; amplify the PB gene serial dilution standards and the Actin gene serial dilution standards, and prepare to make the PB gene and Actin gene standard curves, respectively. The primers, probe sequences and reaction systems for amplifying PB gene and Actin gene are as follows:
PB gene amplification:
PB-F: 5' ggacgagatctacgccttct (SEQ ID NO: 29)
PB-R: 5' ctcatcacgctcacgtacac (SEQ ID NO: 30)PB-probe: 5'tgcgcacggcggtcatcacc (SEQ ID NO: 31)Actin gene amplification:
Actin-F: 5' gggacctgactgactacctc (SEQ ID NO: 32)Actin-R: 5'aatgtcacgcacgatttccc (SEQ ID NO: 33)Actin-probe: 5'caccgagcgcggctacagct (SEQ ID NO:34)

**Table 1: Fluorescence probe quantitative PCR reaction system**

| PB gene amplify reaction system | | Actin gene amplify reaction system | |
|---|---|---|---|
| Reaction reagent | volume | Reaction reagent | volume |
| Realtime PCR Master Mix | 10µl | Realtime PCR Master Mix | 10µl |
| PB-F (10µM) | 0.4µl | Actin-F (10µM) | 0.4µl |
| PB-R (10µM) | 0.4µl | Actin-R (10µM) | 0.4µl |
| PB-probe(10µM) | 0.8µl | Actin-probe(10µM) | 0.8µl |
| Total cellular DNA | 1 µl | Total cellular DNA(10ng-100ng) | 1 µl |
| Water | 7.4µl | Water | 7.4µl |
| Total volume | 20µl | Total volume | 20µl |

4) Real-time fluorescence quantitative PCR was used to amplify the samples to be tested and the standards. The reaction system was shown in Table 1. The reaction program was 1. 50°C for 2min; 2. 95°C for 10min; and 3. 95°C for 15s and 60°C for 1min. A total of 40 cycles were performed. Plot a standard curve, and calculate the amount of residual plasmid according to the standard curve. The formula of calculating copy number is: PB copy number/Actin copy number * 2.

The results are shown in Figure 15. When the amount of electrotransfected plasmid is 5 µg, the copy number of the residual plasmid in the cells decreases drastically with time. On day14 post-transfection (after 3 passages), the average number of residual plasmid copies in each Jurkat cell was less than 10, and the average number of residual plasmid copies in each K562 cell and primary T cell was less than 5; On day20 post-transfection, the average number of residual plasmid copies in each cell in Jurkat cells and K562 cells was less than 1. When the amount of electrotransfected plasmid was reduced to 3 µg, the residual plasmid content in Jurkat cells after electrotransfection was further significantly reduced compared with electrotransfection of 5 µg of plasmid, and the average number of plasmid copies in each cell was less than 10 on day10, and was less than 1 by the 14th day.

The above results show that the vector of the present invention produces a very low residual level in host cells while fully exerting its genome integration function. In the meantime, combined with the results of Example 8, it is also shown that the pKB series vectors of the present invention enables the use of reduced amount of plasmid DNA while ensuring high integration efficiency after electrotransfection, thus further reducing the residual plasmid DNA in cells after electrotransfection.

### Example 10, integration of pKB2003-EGFP vector in cells after electrotransfer

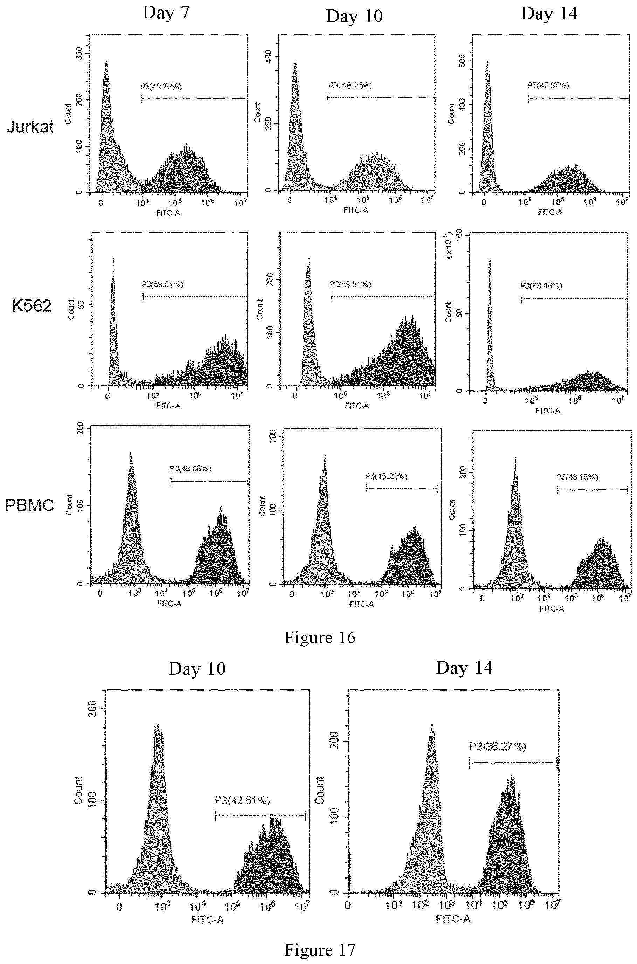

According to the methods described in the above Examples 2 and 4, the pKB2003-EGFP plasmid was electrotransfected into Jurkat cells and K562 cells, respectively, and the positive proportion of the cells was detected by flow cytometry on day7, 10, 14 post-transfection. The results are shown in Figure 16. The positive portion of Jurkat cells electrotransfected with pKB2003-EGFP reached 49% and 48% on day7 and 10, respectively, and remained at a high level close to 48% on day14 after the 3rd passage of cell culture. The positive proportion of K562 cells electrotransfected with pKB2003-EGFP was close to 70% on day7 and 10, and remained at a high level of more than 66% on day14 after the 3rd passage of cell culture.

The above results show that the pKB2003 vector can efficiently integrate and express exogenous genes in cells.

### Example 11, integration in cells after electrotransfection of the pKB series vectors with replaced regulatory elements

According to the methods described in Example 2, 4 and 5, respectively, the amount of plasmid used in the electrotransfection was reduced to 3 µg, and the following vectors were electrotransfected into Jurkat, K562 and PBMC from healthy human blood, respectively: pKB20I1-EGFP, pKB20A1-EGFP, pKB20A1-EGFP, pKB20A2-EGFP, pKB20U1-EGFP, pKB20U2-EGFP, pKB20U3-EGFP, pKB20U4-EGFP, pKB20U5-EGFP, pKB20U6-EGFP, pKB20U7-EGFP, pKB20U8-EGFP, pKB20E1-EGFP, pKB20E2-EGFP, pKB20E3-EGFP, pKB20E4-EGFP, pKB20P1-EGFP, pKB20P2-EGFP, pKB20P3-EGFP, pKB20P4-EGFP, pKB20P5-EGFP and pKB20P6-EGFP. The positive proportion of cells were detected by flow cytometry on day14 post-transfection. The results are shown in Table 2.

**Table 2 The integration rates of the pKB series vectors with replaced regulatory elements in different cells**

| | Jurkat | K562 | Primary T cell |
|---|---|---|---|
| pKB20I1-EGFP | 62.33% | 70.43% | 69.65% |
| pKB20A1-EGFP | 64.02% | 72.33% | 72.55% |
| pKB20A2-EGFP | 68.58% | 69.27% | 70.64% |
| pKB20U1-EGFP | 70.05% | 68.89% | 68.25% |
| pKB20U2-EGFP | 66.51% | 70.97% | 71.36% |
| pKB20U3-EGFP | 62.11% | 73.29% | 72.01% |
| pKB20U4-EGFP | 57.43% | 71.82% | 70.13% |
| pKB20U5-EGFP | 58.27% | 67.44% | 69.81% |
| pKB20U6-EGFP | 61.89% | 69.49% | 67.07% |
| pKB20U7-EGFP | 59.65% | 72.93% | 69.12% |
| pKB20U8-EGFP | 63.43% | 72.01% | 71.79% |
| pKB20E1-EGFP | 62.72% | 66.95% | 65.23% |
| pKB20E2-EGFP | 58.55% | 68.39% | 69.97% |
| pKB20E3-EGFP | 69.79% | 69.28% | 72.17% |
| pKB20E4-EGFP | 71.37% | 71.03% | 70.02% |
| pKB20P 1-EGFP | 66.75% | 72.16% | 70.69% |
| pKB20P2-EGFP | 65.31% | 70.06% | 70.75% |
| pKB20P3-EGFP | 70.03% | 68.24% | 71.53% |
| pKB20P4-EGFP | 59.93% | 69.14% | 68.90% |
| pKB20P5-EGFP | 65.47% | 67.98% | 69.74% |
| pKB20P6-EGFP | 71.01% | 72.09% | 60.58% |

The results in Table 2 show that the above pKB series plasmid vectors with replaced regulatory element sequences can be efficiently integrated into the genomes of different cells, and the integration rates in the above types of cells are at the same level as that of pKB20-EGFP.

### Example 12, Detection of integration efficiency after electrotransfection of pKB205-EGFP vector into primary T cells

According to the method described in Example 5, the amount of plasmid used in electrotransfection was reduced to 3 µg, and pKB205-EGFP was electrotransfected into PBMCs from healthy human blood, and the positive proportions of cells were detected by flow cytometry on day7 and 14 post-transfection.

The results are shown in Figure 17. The positive proportion of T cells transfected with pKB205-EGFP was more than 42% on day7, and remained above 36% on day14 post-transfection (cultured for 3 passages). This shows that pKB205-EGFP vector has higher integration rate and positive expression rate of exogenous genes after being electrotransfected into primary T cells.

### Example 13, the integration site analysis of pKB vector in Jurkat and K562 cells

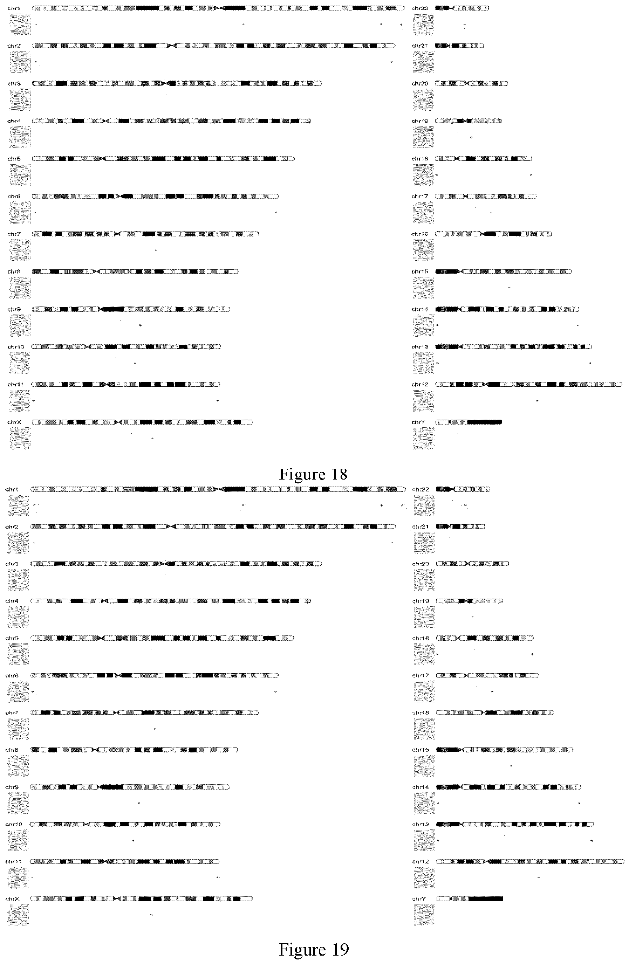

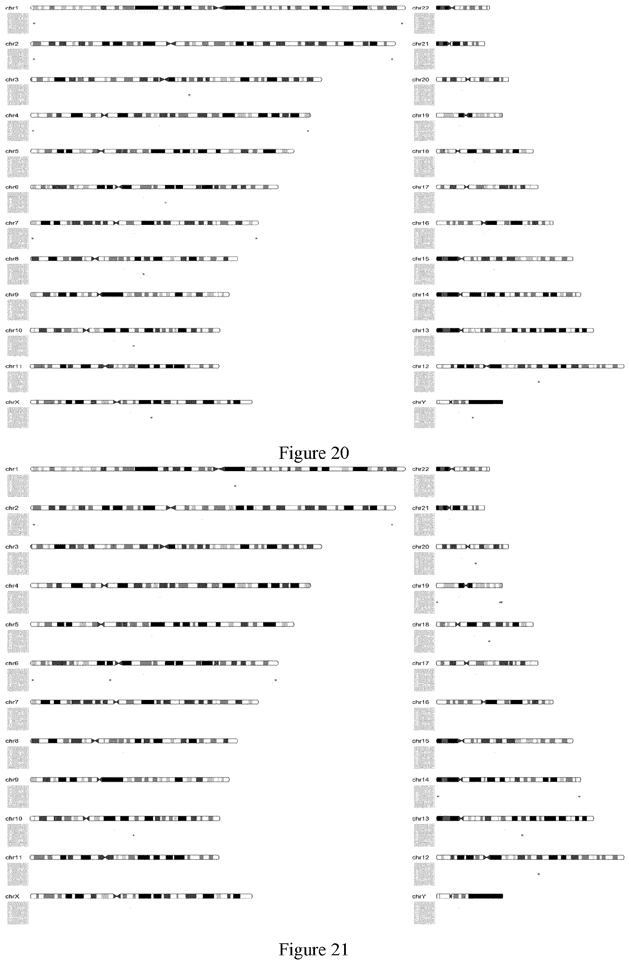

Two groups of Jurkat cells and K562 cells were prepared, respectively, and the pKB20-EGFP plasmid was electrotransfected to these two types of cells according to the methods described in Example 2 and 4. After the electrotransfection, cells were cultured for 14 days and collected for genomic DNA extraction. Whole-genome sequencing was conducted by Genergy Biotechnology (Shanghai) Co., Ltd., and the distribution of EGFP insertion sites in the genome was analyzed. The results are shown in Figure 18-21 and Table 3. Figures 18, 19, 20 and 21 are schematic diagrams of pKB20 vector-mediated genomic integration sites in K562 sample 1, K562 sample 2, Jurkat sample 1 and Jurkat sample 2, respectively.

**Table 3 Classification and statistics of pKB20 vector-mediated integration sites in Jurkat and K562 cells**

| | Downstrea m of gene | intergeni c | Intron | Intron of ncRNA | uptream of gene | 3'UT R | ncRN A Exon of ncRN A | Exon |
|---|---|---|---|---|---|---|---|---|
| K562 sampl e 1 | 4 | 14 | 10 | 4 | 2 | 2 | 0 | 0 |
| K562 sampl e2 | 1 | 11 | 4 | 1 | 1 | 6 | 1 | 0 |
| Jurkat sampl e 1 | 0 | 6 | 2 | 1 | 4 | 0 | 0 | 2 |
| Jurkat sampl e2 | 0 | 7 | 4 | 1 | 0 | 0 | 0 | 4 |

The results in Figures 18-21 and Table 3 show that cell genome integration of the exogenous genes mediated by the pKB20 vector of the present invention mainly occurs in intergenic and intronic regions, and integration sites in exonic regions and gene expression regulation-related regions (such as 3'UTR) are few. This indicates that the integration of exogenous genes in cell genome mediated by pKB20 vector has minor effects on the gene expression of the cell itself.

### Example 14, mRNA expression profile analysis of Jurkat cells and K562 cells after integration of pKB vectors

According to the methods described in the above-mentioned Example 2 and 4, the pKB20-EGFP plasmid was electrotransfected into Jurkat and K562 cells, respectively, and the amount of the plasmid was 4 µg. Cells were harvested 14 days after electrotransfection for mRNA sequencing and expression profile analysis, and the mRNA expression profiles were compared with that of non-transfected Jurkat cells and K562 cells. Two samples of K562 and Jurkat cells each were used for analysis.

The results of sequencing and analysis showed that compared with the non-transfected control cells, the mRNA expression of genes adjacent to the pKB20 integration sites after electrotransfection of pKB20-EGFP exhibited minor alteration, and the differential expression of related genes is as shown in Table 4-7. This shows that the integration of the pKB vector of the present invention into the genome has minor influence on cellular genome stability and gene expression profile.

**Table 4 Differential expression of genes adjacent to the integration site of K562 sample 1**

| related gene name | insertion site location | differentially expressed or not |
|---|---|---|
| DENND1B;C1orf53 | intergenic | No |
| GNPAT;EXOC8 | intergenic | No |
| ELK4 | uptream of the gene | downregulation |
| MTRNR2L5;ZWINT | intergenic | No |
| LPXN | downstream of the gene | No |
| CCDC 179;MIR8054 | intergenic | No |
| LINC01995;ATP 11B | intergenic | No |
| LINC01267 | uptream of the gene | No |
| NFKB1 | downstream of the gene | No |
| LCORL;SLIT2 | downstream of the gene | No |
| LINC01378;LINC02264 | intergenic | No |
| LINC01378;LINC02264 | intergenic | No |
| ATP10B | downstream of the gene | No |
| CRHBP;AGGF1 | intergenic | No |
| CRHBP;AGGF1 | intergenic | No |
| TULP1;FKBP5 | intergenic | No |
| ADGB | downstream of the gene | No |
| LOC105374972;NRSN1 | intergenic | No |
| LMX1B;ZBTB43 | intergenic | No |
| PCDH11X;MIR4454 | intergenic | No |

**Table 5 Differential expression of genes adjacent to the integration site of K562 sample 2**

| Related gene name | insertion site location | differentially expressed or not |
|---|---|---|
| TSPAN2 | uptream of the gene | No |
| KHDRBS1 | downstream of the gene | No |
| LINC00900;LOC 101929011 | intergenic | No |
| LINC01309;DAOA-AS1 | intergenic | No |
| KIF2B;TOM1L1 | intergenic | No |
| MIB1;GATA6-AS1 | intergenic | No |
| TWSG1;RALBP1 | intergenic | No |
| MCM5;RASD2 | intergenic | No |
| MIR4465;NMBR | intergenic | No |
| FNDC 1 ;LOC 1 02724053 | intergenic | No |
| MGC4859;NDUFA4 | intergenic | No |
| STOM;GGTA1P | intergenic | No |
| FAM9A;FAM9B | intergenic | No |

**Table 6 Differential expression of genes adjacent to the integration site of Jurkat sample 1**

| Related gene name | insertion site location | differentially expressed or not |
|---|---|---|
| TMEM167B | uptream of the gene | No |
| LOC441666 | uptream of the gene | No |
| LINC01831;LOC 100287010 | uptream of the gene | No |
| PLSCR5;LINC02010 | intergenic | No |
| MIR7641-2;KIAA1211 | intergenic | No |
| CD83 | uptream of the gene | No |
| LINC00972;GRM3 | intergenic | No |
| STC1;ADAM28 | intergenic | No |
| GPR174;ITM2A | intergenic | No |
| NLGN4Y;NONE | intergenic | No |

**Table 7 Differential expression of genes adjacent to the integration site of Jurkat sample 2**

| Related gene name | insertion site location | differentially expressed or not |
|---|---|---|
| SFRP5;LINC00866 | intergenic | No |
| LINC00558;LINC00458 | intergenic | No |
| LINC01551;PRKD1 | intergenic | No |
| EGLN3;SPTSSA | intergenic | No |
| NFATC1;LOC284241 | intergenic | No |
| IPO5P1;ZNF91 | intergenic | No |
| AP2S1;ARHGAP35 | intergenic | No |

### Example 15, Preparation of pKB20-HER2CAR Vector and HER2CAR-T Cells

The EF1A promoter sequence (SEQ ID NO: 11) with NFAT motif was inserted between the XbaI and EcoRI sites of the multiple cloning site of pKB20, and the coding sequence of HER2CAR (SEQ ID NO: 43) was inserted between the EcoRI and SalI sites. The obtained vector is named pKB20-HER2CAR. The EF1A promoter sequence with NFAT motif and the coding sequence of HER2CAR were synthesized by Shanghai Generay Biotechnology Co., Ltd.

PBMCs isolated from peripheral blood were electrotransfected with the pKB20-HER2CAR vector according to the following steps to prepare HER2-targeting CAR-T cells. The PBMCs used were purchased from AllCells and derived from the peripheral blood of healthy adults.
1) Collect the suspended cells in a 50ml centrifuge tube, centrifuge at 1200rmp for 3min;
2) Discard the supernatant, resuspend cells in normal saline, centrifuge at 1200rmp for 3min, discard the normal saline, and repeat this step followed by cell counts;
3) Take two 1.5ml centrifuge tubes, add 5×10⁶ cells to each tube, and centrifuge at 1200rmp for 3min;
4) Discard the supernatant, use a electrotransfection kit (purchased from Lonza), add 18 µL of solution I, 82 µL of solution II, and 5 µg of pKB20 plasmid into the first tube as control, and add 5 µg of pKB20-HER2CAR plasmid into the second tube;
5) Transfer the cell suspension mixed with the plasmid in the centrifuge tube to the electroporation cuvette, put it into the electroporator, select program T020, and apply electroporation;
6) Use the micropipette in the kit to transfer the electrotroporated cell suspension into the 12-well plate (AIM-V culture medium containing 2% FBS), mix, and culture in a 37°C 5% CO₂ incubator; meanwhile, coat 2 wells of a 6-well plate with a mixture containing 5 µg/mL HER2 extracellular region antigen (SinoBiological 10004-H08H) and 5 µg/mL CD28 antibody (ThermoFisher 14-0281-82), add 1mL to each well, and incubate the 6-well plate at 37°C.
7) After 6 hours, transfer the electroporated cells cultured in the 37°C, 5% CO₂ incubator into the 6-well plate coated with HER2 extracellular domain antigen and CD28 antibody, add IL-2 to a final concentration of 100 IU/mL, and add culture medium to a final volume of 3ml. After 4-5 days of culture, observe the growth of T cells, transfer the activated cells into AIM-V medium containing 2% FBS and continue to culture until the required cell amount is reached and collect HER2CAR-T cells; cells transfected with pKB20 empty vectors are Mock-T cells and serve as control.

### Example 16, Detection of CAR-positive cells in HER2CAR-T cells

1) 1×10⁶ HER2CAR-T cells prepared in Example 15 were collected and centrifuged at 1000rpm for 3min;
2) The supernatant was discarded, normal saline was added to resuspend the cells, and cells were centrifuged at 1000rpm for 3min;
3) The supernatant was discarded, 100 µL of normal saline was added to resuspend the cells, and 1 µL of biotin-labeled HER2 antigen (purchased from KactusBiological with Product Number: HER-HM402) was added to each tube. The tubes were incubated at 4°C for 30 minutes;
4) Appropriate amount of normal saline was added, cells were centrifuged at 1000rpm for 3min, washed twice, and the supernatant was discarded;
5) 100 µL of normal saline to was added to resuspend the cells. 1 µL of PE-labeled streptavidin (purchased from ThermoFisher with product number: S20982) was added, mixed well, and incubated at 4°C for 30 min;
6) Appropriate amount of normal saline was added, cells were centrifuged at 1000rpm for 3min, washed twice, and the supernatant was discarded;
7) Cells were resuspended with 400 µL of normal saline, and submitted to flow cytometry.

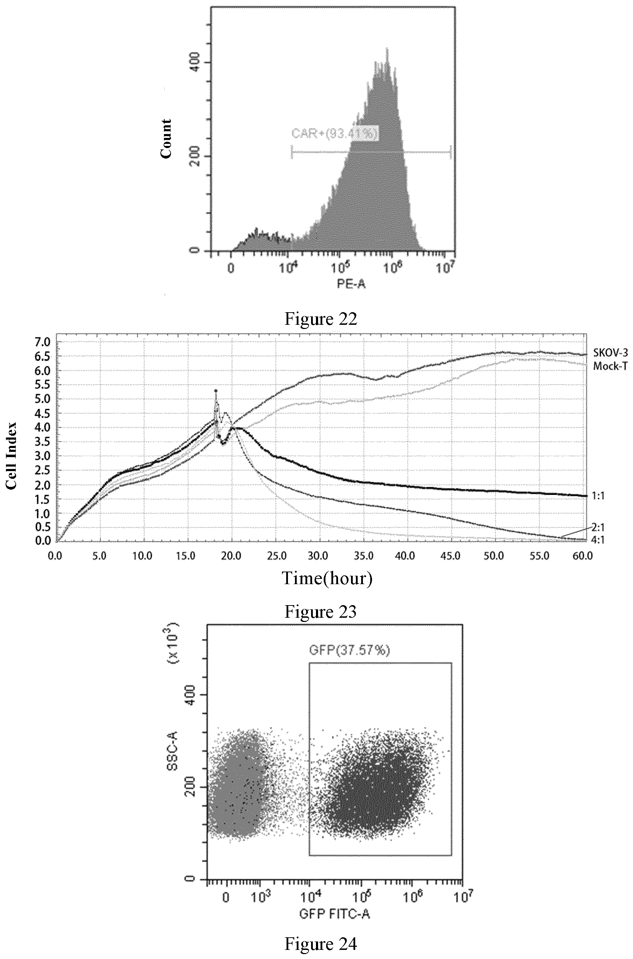

The results are shown in Figure 22. Up to 93.41% of the cells were PE-positive, indicating that the proportion of CAR-positive cells in the HER2CAR-T cells prepared by electrotransfecting pKB20-HER2CAR plasmid into PBMCs was over 90%.

### Example 17, Cell killing function test of HER2-CAR-T

The in vitro killing activity of the HER2CAR-T cells obtained in Example 15 was detected using the real-time label-free cell function analyzer (RTCA) of ACEA Biosciences Inc., and the specific steps were as follows:
(1) Zero adjustment: 50 µL of DMEM culture solution was added to each well, put into the instrument,and step 1 was selected and zero adjustment was performed;
(2) Plating of target cells: human ovarian cancer cells SKOV-3 (purchased from ATCC, HER2 positive) were plated onto a plate containing detection electrodes at 10⁴ cells/50 µL per well, and stood for several minutes. When the cells became stably attached, they were mounted onto the instrument, and step 2 was initiated to cultivate the cells;
(3) Adding effector cells: after the target cells were cultured for 18 hours, control cells Mock-T and effector cells HER2CAR-T were added, respectively, the cell suspension volume being 50 µL per well. Three effector-target ratios, 1:1, 2:1, and 4:1 were set for HER2CAR-T, respectively, and one effector-target ratio 4:1 set for Mock-T.The effector-target ratio is calculated according to the total number of viable cells. Co-culture was started, and the cell proliferation curve after 60 hours was monitored.

The results are shown in Figure 23. The killing curve of the control Mock-T cell group is close to the change of the SKOV-3 tumor cell curve, indicating that the killing effect of Mock-T cells on SKOV-3 cells is minor. The killing effects of HER2CAR-T on SKOV-3 cells are very obvious at all three effector-target ratios, 1:1, 2:1, and 4:1, and also is significantly improved with the increase of the effector-target ratio.

### Example 18, Preparation of pKB20-NY-ESO-1-TCR vector and NY-ESO-1 TCR-T cells

### Construction of pKB20-NY-ESO-1-TCR vector:

The gene DNA sequence encoding the α chain and β chain of the TCR that recognizes the NY-ESO-1 antigen peptide SLLMWITQC (HLA-*02:01) was synthesized, and the two chains weree linked by the DNA sequence encoding the P2A peptide. the spliced sequence is as shown in SEQ ID NO:44. Then, the DNA sequence encoding EGFP was connected to the 3' end of SEQ ID NO:44 by the DNA encoding the P2A peptide to obtain the NY-ESO-1-TCR gene covalently linked to the EGFP reading frame. The obtained sequence is as shown in SEQ ID NO :45. The EF1A promoter sequence (SEQ ID NO: 11) with NFAT motif was inserted between the XbaI and EcoRI sites of the multiple cloning site of pKB20, and the coding sequence of the NY-ESO-1-TCR gene (SEQ ID NO:45) covalently linked to EGFP reading frame was inserted between the EcoRI and SalI sites. The vector obtained was named pKB20-NY-ESO-1-TCR. The EF1A promoter sequence with the NFAT motif and the coding sequence of the NY-ESO-1-TCR gene covalently linked to the EGFP reading frame were synthesized by Shanghai Generay Biotechnology Co., Ltd.Preparation of

### NY-ESO-1 TCR-T cells:

The 6-well plate was coated with a coating solution containing 5 µg/ml anti-CD3 antibody (ThermoFisher 14-0037-82) and 5 µg/ml anti-CD28 antibody (ThermoFisher 14-0281-82) at room temperature for 2-4 hours. The coating solution was discarded, and the plate was washed with normal saline for 1-3 times, and AIM-V medium containing 2% FBS was added for use; human peripheral blood PBMC (HLA-*02:01, purchased from ALLCELLS) were recovered in a 37°C water bath, and adherently cultured for 2-4 hours. The suspended cells that did not attach were the initial T cells. The suspended cells were collected into a 15ml centrifuge tube, centrifuged at 1200rmp for 3min. The supernatant was discarded, normal saline was added, and cells were centrifuged at 1200rmp for 3min. The normal saline was discard, and this step was repeated; the washed initial T cells were transferred to the antibody-coated wells filled with ready-to-use medium, cultured at 37°C, 5% CO₂ for 3 to 4 days, and submitted to subsequent experiments.

Preparation of NY-ESO-1 TCR-expressing T cells by electrotransfection. The steps are as follows
1) AIM-V medium was added to 2 of the wells of a 12-well plate in advance, 2 mL per well, and then the plated was transferred to a cell culture incubator at 37°C with 5% CO₂ for one-hour preheating;
2) The electrotransfection solution for each well in a single experiment was prepared according to the following table 8, and 2 wells were prepared:

**Table 8**

| | 100 µL Nucleocuvette^{™} Strip (µL) |
|---|---|
| Volume of Nucleofector^{™} solution | 82 |
| Electrotransfection Supplementary Solution | 18 |

3) The obtained activated T cells were transferred into two EP tubes, 5×10⁶ cells were added into each EP tube, and centrifuged at 1200 rpm for 5 min. The supernatant was discarded, and the cells were resuspended with 500 µL of normal saline. The centrifugation steps were repeated to wash the cell pellet;
4) 4 µg of the plasmids pKB20-NY-ESO-1-TCR and pKB20-EGFP vector each was added to the electrotransfection mixture in the two wells prepared in 2), and then the mixture stood at room temperature for 30 minutes;
5) The two tubes of activated T cells were resuspended by the electrotransfection solution containing the plasmids prepared in 4), 100 µL in each tube. The cell resuspension was carefully pipetted the into a LONZA 100 µL electrotroporation cuvette, and then the cuvette was placed into the LONZA Nucleofector^{™} 2b electroporation chamber. The electroporation program, for which T-020 was selected, was executed ;
6) When the electroporation was complete, electroporation cuvettes were removed with care. The cell suspension was transferred to EP tubes, 200 µL of preheated AIM-V medium was added to each tube, and then the cells were transferred to the wells of the 12-well plate in 1) containing preheated AIM-V culture and cultured at 37°C and 5%CO₂. After the cells were cultured for 1 h, the compound G150 (purchased from MedChemExpress) was added at a final concentration of 5 µM, and then the cells were cultured for 13 days, during which the cells were passaged according to the cell proliferation status. After 13 days, each electrotransfected sample was submitted to total cell count and cell viability test, thus generating the NY-ESO-1 TCR-T cells and Mock-T cells, respectively.

Example 19, NY-ESO-1 TCR expressing positivity test for NY-ESO-1 TCR-T cells
1) 1×10⁶ NY-ESO-1 TCR-T cells prepared in Example 18 was collected and centrifuged at 1000rpm for 3min;
2) The supernatant was discarded, and normal saline was added to resuspend the cells. Cells were then centrifuged at 1000rpm for 3min;
3)Cells were submitted to flow cytometry assay.

The results are shown in Figure 24. The proportion of cells positive for EGFP expression was 37.57%. The gene DNA sequence of α chain and β chain of NY-ESO-1 TCR and the coding DNA sequence of EGFP are linked by the P2A peptide coding sequence, and the cells with positive expression of EGFP can indirectly reflect the expression of NY-ESO-1 TCR gene. It can be speculated that the proportion of cells positive for NY-ESO-1 TCR expression is about 37%.

### Example 20, Cell killing function test of NY-ESO-1 TCR-T

The in vitro killing activity of the NY-ESO-1 TCR-T cells obtained in Example 18 was detected using the real-time label-free cell function analyzer (RTCA) of ACEA Biosciences Inc., and the specific steps are as follows:
(1) Zero adjustment: 50 µL of DMEM culture solution was added to each well before being put into the instrument. Step 1 was selected, and zero adjustment conducted;
(2) Target cell plating: human malignant melanoma cell line A375 (purchased from ATCC, American Culture Collection Center, positive for NY-ESO-1 expression) cells were plated onto a plate containing detection electrodes at a concentration of 10⁴ cells/50 µL per well, and stood for several minutes. When the cells became stably attached, they were mounted onto the instrument, and step 2 was initiated to cultivate the cells;(3) Adding effector cells: After the target cells were cultured for 18 hours, the cell index was checked. When the cell index value reached 1, the control cells Mock-T and the effector cells NY-ESO-1 TCR-T were added, respectively, 50 µL per well. Two effector-target ratios, 0.25: 1 and 0.5:1, were set for NY-ESO -1TCR-T, and one effect-target ratio 0.5:1 set for Mock-T. The effector-target ratio was calculated according to the number of positive cells expressing NY-ESO-1 TCR. Step 3 co-cultivation was initiated, and the cell proliferation curve after 70 hours was monitored.

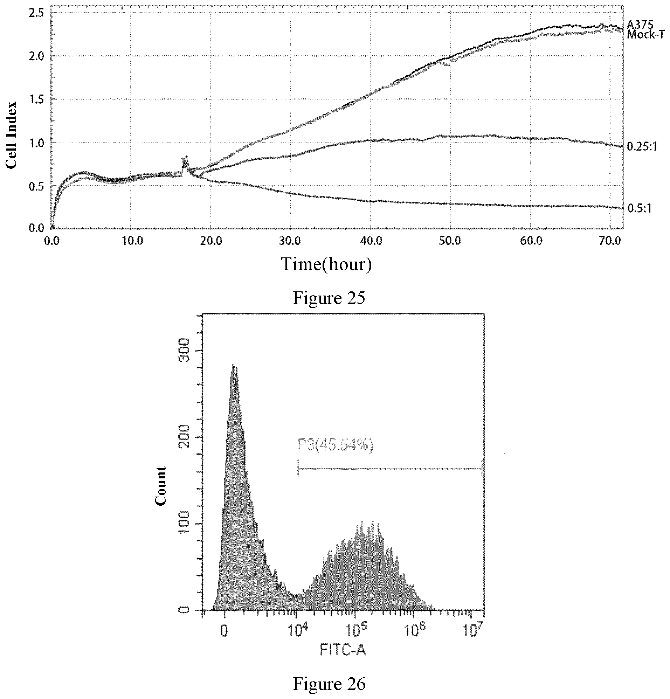

The results are shown in Figure 25. The killing curve of the control Mock-T cell group basically overlaps with the A375 tumor cell curve, indicating that the Mock-T cells basically have no killing effect on A375 cells. The killing effects of NY-ESO-1 TCR-T on A375 cells are very obvious at both the effector-target ratios 0.25:1 and 0.5:1, and also are significantly improved with the increase of the effector-target ratio.

Comparative example 1, integration of pNB vector containing EGFP expression cassette in K562 cellsThe pNB vector and pNB328-EGFP were constructed according to the methods described in Example 1 on page 15 of the specification of Chinese patent CN105154473B and Example 2 on page 16 of the specification, respectively. According to the method described in Example 4 of the present application, pNB328-EGFP was used to prepare K562 stably integrated with and expressing EGFP by electrotransfection, and cells positive for EGFP expression were detected by flow cytometry on day 14 post-transfection (cultured for 3 passages).

The results are shown in Figure 26, 14 days after electrotransfection, the proportion of K562 cells positive for EGFP expression was 45.54%.

### Comparative Example 2, the integration of pNB vector containing EGFP expression cassette in primary T cells

According to the method described in Example 5 of the present application, pNB328-EGFP was used to prepare primary T cells stably integrated with and expressing EGFP by electrotransfection, and cells positive for EGFP expression were detected by flow cytometry on day 14 post-transfection (cell culture 3 generations). The PBMCs used for electrotransfection are the same batch of PBMCs as that used in Example 5. 14 days after electrotransfection (cultured for 3 passages), flow cytometry was used to detecte cells positive for EGFP expression.

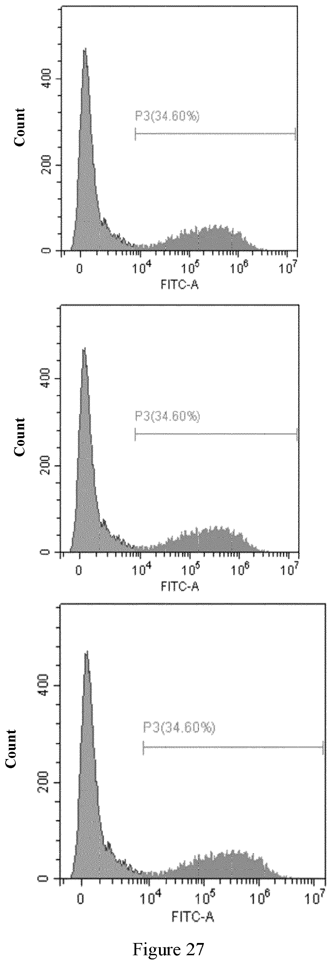

The results are shown in Figure 27. 14 days after electrotransfection, the proportion of T cells positive for EGFP expression was 34.60%.

Although specific embodiments of the present invention have been described in detail, those skilled in the art will understand, based on all the teachings that have been disclosed, various modifications and substitutions can be made to details, and these changes are all within the scope of the invention. The full scope of the invention is given by the appended claims and any equivalents thereof.

## Claims

1. A nucleic acid construct, comprising or consisting of the following elements: a transposon 3' terminal repeat sequence, a first polyA sequence, an insulator sequence with transcription termination function, a transposon 5' terminal repeat sequence,
preferably, the nucleic acid construct further comprises one or more elements selected from the group consisting of a transposase coding sequence, a promoter controlling the expression of the transposase, a multiple cloning insertion site, an enhancer, a 5'UTR, a second polyA sequence and an exogenous gene of interest,
preferably, any one or more of the transposase coding sequence, the promoter controlling the expression of the transposase, the 5'UTR and the second polyA sequence are outside the region between the transposon 3' terminal repeat sequence and the transposon 5' terminal repeat sequence.

2. The nucleic acid construct according to claim 1, wherein the nucleic acid construct comprises the following elements: a transposon 3' terminal repeat sequence, a first polyA sequence, an insulator sequence with transcription termination function, a transposon 5' terminal repeat sequence, a transposase encoding sequence and a promoter controlling the expression of the transposase,
preferably, the nucleic acid construct further comprises one or more elements selected from the group consisting of: a multiple cloning insertion site, an enhancer, a 5'UTR, a second polyA sequence and an exogenous gene of interest.

3. The nucleic acid construct according to claim 2, wherein,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a transposase coding sequence and a promoter for controlling transposase expression,
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a transposase coding sequence and a promoter for controlling transposase expression,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a transposase coding sequence, 5'UTR and a promoter for controlling transposase expression,
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a transposase coding sequence, a 5'UTR and a promoter for controlling transposase expression,
the nucleic acid construct comprises sequentially: a transposon 3' terminal repeat sequence, a first polyA sequence, an insulator sequence with transcription termination function, a transposon 5' terminal repeat sequence, a promoter for controlling transposase expression, a transposase coding sequence and a second polyA sequence,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a promoter for controlling transposase expression, a transposase coding sequence and a second polyA sequence,
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a promoter for controlling transposase expression, a transposase coding sequence and a second polyA sequence,
the nucleic acid construct comprises sequentially: a transposon 3' terminal repeat sequence, a multiple cloning insertion site, a first polyA sequence, an insulator sequence with transcription termination function, a transposon 5' terminal repeat sequence, a promoter for controlling transposase expression, a 5'UTR, a transposase coding sequence and a second polyA sequence,
the nucleic acid construct comprises sequentially: a transposon 3' terminal repeat sequence, a multiple cloning insertion site, a first polyA sequence, an enhancer, an insulator sequence with transcription termination function, a transposon 5' terminal repeat sequence, a promoter for controlling transposase expression, a 5'UTR, a transposase coding sequence and a second polyA sequence,
the nucleic acid construct comprises sequentially: a transposon 3' terminal repeat sequence, an insulator sequence with transcription termination function, a multiple cloning insertion site, a first polyA sequence, a transposon 5' terminal repeat sequence, a promoter for controlling transposase expression, a 5'UTR, a transposase coding sequence and a second polyA sequence,
the nucleic acid construct comprises sequentially: a transposon 3' terminal repeat sequence, an insulator sequence with transcription termination function, a multiple cloning insertion site, a first polyA sequence, en enhancer, a transposon 5' terminal repeat sequence, a promoter for controlling transposase expression, a 5'UTR, a transposase coding sequence and a second polyA sequence, or
the nucleic acid construct comprises sequentially: a transposon 3' terminal repeat sequence, an enhancer, an insulator sequence with transcription termination function, a multiple cloning insertion site, a first polyA sequence, a transposon 5' terminal repeat sequence, a promoter for controlling transposase expression, a 5'UTR, a transposase coding sequence and a second polyA sequence.

4. The nucleic acid construct according to any one of claims 1-3, wherein the nucleic acid construct has one or more characteristics selected from the following:
the orientation of the expression cassette of the transposase is the same as or opposite to the orientation of the expression cassette of the exogenous gene,
the orientation of the expression cassette of the transposase is the same as or opposite to the orientation of the sequence between the transposon 3' terminal repeat sequence and the transposon 5' terminal repeat sequence,
the position of the repeat sequence of the 5' end of the transposon and the position of the transposon 3' terminal repeat sequence is interchangable,
the transposon 3' terminal repeat sequence is the PiggyBac transposon 3' terminal repeat sequence,
the transposon 5' terminal repeat sequence is the PiggyBac transposon 5' terminal repeat sequence,
the enhancer is selected from the group consisting of CMV enhancer sequence, SV40 enhancer, human epsilon globin 5' HS2 enhancer, and chicken β globin gene 5' HS4 enhancer,
the transposase is PiggyBac transposase,
the 5'UTR is selected from the group consisting of: 5'UTR of C3 gene, ORM1 gene, HPX gene, FGA gene, AGXT gene, ASL gene, APOA2 gene, and ALB gene,
the promoter is selected from the group consisting of CMV promoter, miniCMV promoter, CMV53 promoter, miniSV40 promoter, miniTK promoter, MLP promoter, pJB42CAT5 promoter, YB_TATA promoter, EF1α promoter, SV40 promoter, UbiquitinB promoter, CAG promoter, HSP70 promoter, PGK-1 promoter, β-actin promoter, TK promoter and GRP78 promoter,
the transposase coding sequence contains or is operably linked to a single copy or multiple copies of a nuclear localization signal coding sequence.

5. The nucleic acid construct according to any one of claims 1-3, wherein the nucleic acid construct has one or more characteristics selected from the following:
the nucleotide sequence of the transposon 3' terminal repeat sequence is as shown in SEQ ID NO: 1,
the nucleotide sequence of the transposon 5' terminal repeat sequence is as shown in SEQ ID NO: 6,
the sequence of the multiple cloning insertion site is as shown in SEQ ID NO: 2,
the first polyA sequence is as shown in SEQ ID NO: 3, 13 or 16,
the second polyA sequence is as shown in SEQ ID NO: 3, 13 or 16,
the enhancer sequence is as shown in any one of SEQ ID NO:4, 26-28.
the insulator sequence is as shown in SEQ ID NO: 5 or 15,
the amino acid sequence of the PiggyBac transposase is as shown in SEQ ID NO:36; preferably, the coding sequence of the PiggyBac transposase is as shown in SEQ ID NO:7,
the 5'UTR sequence is as shown in any one of SEQ ID NO:8, and 17-24,
the sequence of the promoter is as shown in any one of SEQ ID NO:9, SEQ ID NO:37-42,
the nuclear localization signal is a c-myc nuclear localization signal; preferably, the nuclear localization signal has the sequence shown in SEQ ID NO:35.

6. The nucleic acid construct according to any one of claims 1-3, wherein,
the nucleic acid construct comprises a sequence as shown in SEQ ID NO: 10 or 14, or
the nucleic acid construct is a recombinant vector, preferably, the nucleic acid construct is a recombinant cloning vector or a recombinant expression vector.

7. A host cell comprising
(1) the nucleic acid construct according to any one of claims 1-6, and/or
(2) the sequence between the transposon 3' terminal repeat sequence and the transposon 5' terminal repeat sequence of the nucleic acid construct according to any one of claims 1-6,
preferably, the host cell is a mammalian cell,
more preferably, the host cells are selected from the group consisting of: T cells, Jurkat cells, K562 cells, embryonic stem cells, tumor cells, HEK293 cells and CHO cells.

8. A pharmaceutical composition, comprising the nucleic acid construct according to any one of claims 1-6 or the host cell according to claim 8 and pharmaceutically acceptable excipients.

9. Use of the nucleic acid construct according to any one of claims 1-6 or the host cell according to claim 7 in the manufacture of or as a medicament, reagent or tool, wherein the medicament, reagent or tools are used for the integration of exogenous gene expression cassettes into the genome of target cells, or for gene therapy, cell therapy, stem cell induction or differentiation,
preferably, the host cell is a mammalian cell,
more preferably, the target cells are selected from the group consisting of immune cells, Jurkat cells, K562 cells, embryonic stem cells, tumor cells, HEK293 cells and CHO cells,
further preferably, the immune cells are selected from any one or more of the group consisting of T cells, B cells, CIK cells, LAK cells, NK cells, cytotoxic T lymphocytes (CTL), dendritic cells (DC), tumor infiltrating lymphocytes (TIL), macrophages, NK T cells, and γδT cells.

10. A method for integrating an exogenous gene or its expression cassette into the genome of a cell, comprising introducing into said cells the nucleic acid construct according to any one of claims 1-6 containing the exogenous gene and optionally its promoter, and optionally incubating said cells under conditions in which a transposase integrates the exogenous gene or its expression cassette into the genome of the cell, said exogenous gene and optionally its promoter are located in the multiple cloning insertion site of said nucleic acid construct,
preferably, the transposase is PiggyBac transposase.
